(19) Europäisches Patentamt
European Patent Office
Office européen des brevets

(11) EP 2 948 130 B1

(12) **EUROPEAN PATENT SPECIFICATION**

(45) Date of publication and mention of the grant of the patent:
**13.03.2019 Bulletin 2019/11**

(21) Application number: **14704453.1**

(22) Date of filing: **24.01.2014**

(51) Int Cl.:
*A61K 9/00* (2006.01) *A61K 47/32* (2006.01)
*A61K 31/64* (2006.01) *A61P 27/02* (2006.01)

(86) International application number:
**PCT/US2014/013036**

(87) International publication number:
**WO 2014/117010 (31.07.2014 Gazette 2014/31)**

(54) **COMPOSITION FOR OPHTHALMIC ADMINISTRATION**

ZUSAMMENSETZUNG ZUR VERABREICHUNG IN DIE AUGEN

COMPOSITION POUR ADMINISTRATION OPHTALMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.01.2013 US 201361756306 P**

(43) Date of publication of application:
**02.12.2015 Bulletin 2015/49**

(73) Proprietor: **Rigel Pharmaceuticals, Inc.**
**South San Francisco, CA 94080 (US)**

(72) Inventors:
- **REN, Hong**
  **San Jose, CA 95135 (US)**
- **SUN, Thomas**
  **Palo Alto, CA 94306 (US)**

(74) Representative: **J A Kemp**
**14 South Square**
**Gray's Inn**
**London WC1R 5JJ (GB)**

(56) References cited:
**WO-A1-2012/012476 US-A1- 2004 198 829**
**US-A1- 2011 028 503**

- **DESAI S D ET AL: "IN VITRO EVALUATION OF PLURONIC F127-BASED CONTROLLED-RELEASE OCULAR DELIVERY SYSTEMS FOR PILOCARPINE", JOURNAL OF PHARMACEUTICAL SCIENCES, AMERICAN PHARMACEUTICAL ASSOCIATION, WASHINGTON, US, vol. 87, no. 2, 1 February 1998 (1998-02-01), pages 226-230, XP000729422, ISSN: 0022-3549, DOI: 10.1021/JS970090E**
- **H FORTTMANN AND A QUADIR: "Polyvinylpyrrolidone (PVP) - One of the most widely used excipients in pharmaceuticals: an overview", DRUG DELIVERY TECHNOLOGY, vol. 8, no. 6, 1 June 2008 (2008-06-01), pages 22-27, XP002722418,**
- **WALTER R. WASYLASCHUK ET AL: "Evaluation of Hydroperoxides in Common Pharmaceutical Excipients", JOURNAL OF PHARMACEUTICAL SCIENCES, vol. 96, no. 1, 1 January 2007 (2007-01-01), - 1 January 2007 (2007-01-01), pages 106-116, XP055281352, WASHINGTON, US ISSN: 0022-3549, DOI: 10.1002/jps.20726**
- **ISP: "Plasdone Povidones Product overview", INTERNET CITATION, 1 April 2010 (2010-04-01), pages 1-4, XP002672586, Retrieved from the Internet: URL:http://www.isppharmaceuticals.com/Literature/ISP-PH5768-Plasdone-C-K-Povidones-Sheet-VF.pdf [retrieved on 2010-04-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **Anonymous: "Povidone", Martindale: The Complete Drug Reference, 10 January 2017 (2017-01-10), pages 1-5, XP055376411, Retrieved from the Internet: URL:https://www.medicinescomplete.com/mc/martindale/current/ms-5450-e.htm?q=povidone&t=search&ss=text&tot=688&p=1#_hit [retrieved on 2017-05-29]**

Remarks:

The file contains technical information submitted after the application was filed and not included in this specification

## Description

## FIELD

The present disclosure relates to embodiments of a composition suitable for ophthalmic administration and methods of making and using the composition to treat an eye disease or disorder.

## BACKGROUND

JAK kinases (JAnus Kinases) are a family of cytoplasmic protein tyrosine kinases including JAK1, JAK2, JAK3 and TYK2. Each of the JAK kinases is selective for the receptors of certain cytokines, though multiple JAK kinases may be affected by particular cytokine or signaling pathways. Studies suggest that JAK3 associates with the common gamma ($\gamma$c) chain of the various cytokine receptors. JAK3 in particular selectively binds to receptors and is part of the cytokine signaling pathway for IL-2, IL-4, IL-7, IL-9, IL-15 and IL-21. JAK1 interacts with, among others, the receptors for cytokines IL-2, IL-4, IL-7, IL-9 and IL-21, while JAK2 interacts with, among others, the receptors for IL-9 and TNF-$\alpha$. Upon binding of certain cytokines to their receptors (for example, IL-2, IL-4, IL-7, IL-9, IL-15 and IL-21), receptor oligomerization occurs, resulting in the cytoplasmic tails of associated JAK kinases being brought into proximity and facilitating the trans-phosphorylation of tyrosine residues on the JAK kinase. This trans-phosphorylation results in the activation of the JAK kinase.

Phosphorylated JAK kinases bind various STAT (Signal Transducer and Activator of Transcription) proteins. STAT proteins, which are DNA binding proteins activated by phosphorylation of tyrosine residues, function both as signaling molecules and transcription factors and ultimately bind to specific DNA sequences present in the promoters of cytokine-responsive genes. JAK/STAT signaling has been implicated in the mediation of many abnormal immune responses such as allergies, asthma, autoimmune diseases such as transplant (allograft) rejection, rheumatoid arthritis, amyotrophic lateral sclerosis and multiple sclerosis, eye diseases or disorders, as well as in solid and hematologic malignancies such as leukemia and lymphomas.

Diseases and/or disorders of the eye (*e.g.*, keratitis sicca or keratoconjunctivitis sicca) represent a particularly widespread clinical problem. Dry eye syndrome is generally caused by either evaporative dysfunction or tear-deficiency. The evaporative dysfunctions occur in the presence of normal lacrimal function, but may be caused by meibomian gland dysfunction, loss of normal eyelid function, or ocular surface causes (such as contact lens use or ocular allergy). Tear deficiencies may be caused by Sjögren’s syndrome, but non-Sjögren’s disorders also may result in tear deficiencies.

A common cause of dry eye symptoms is Sjögren’s syndrome, which is an autoimmune disorder whereby immune cells attack and impair the glands that produce tears and saliva. The hallmark symptoms of the disorder are dry mouth and dry eyes, but the symptoms are not so limited, as Sjögren’s syndrome is known to cause general dryness. Sjögren’s syndrome affects 1-4 million people in the United States, with women being nine times more likely to develop the disease. Sjögren’s syndrome can occur as a primary condition or as a secondary disorder in association with other autoimmune diseases, such as systemic lupus erythematosus ("lupus") or rheumatoid arthritis. Dry eye symptoms are frequently seen in association with other common disorders, such as rosacea. Dry eye symptoms are also a common side-effect of many medications, such as isotretinoin, diuretics, tricyclic antidepressants, sedatives, antihypertensive medications, oral contraceptives, antihistamines, nasal decongestants, and many others.

US 2011/028503 A1 discusses compounds I and II disclosed herein and compositions comprising compounds I and II.

## SUMMARY

The present invention provides a composition for ophthalmic administration, comprising:

(i) 5% w/v to 15% w/v polyvinylpyrrolidone having an average molecular weight of from 40,000 to 60,000, and less than 400 ppm peroxide; and

(ii) at least one compound selected from the group consisting of

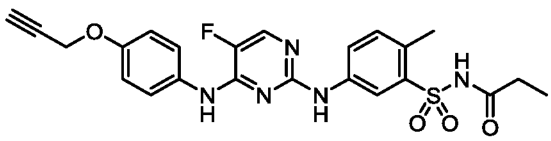

,

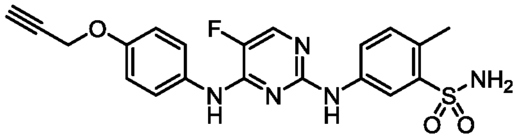,

and pharmaceutically acceptable salts, solvates, or *N*-oxides thereof.

Disclosed herein are embodiments which concern a composition for ophthalmic administration. In particular disclosed embodiments of the present invention, the polyvinylpyrrolidone is present in an amount ranging from 5% w/v to 15% w/v, such as 5% w/v to about 10% w/v. An exemplary amount of the polyvinylpyrrolidone that may be used in the disclosed composition is about 7.5% w/v. In disclosed embodiments of the invention, the polyvinylpyrrolidone contains less than about 400 ppm peroxide and has an average molecular weight of from about 40,000 to at least about 60,000.

Embodiments of the disclosed composition can further comprise one or more additional components, such as a buffer and a tonicity agent. An exemplary buffer is a phosphate buffer, such as monobasic sodium phosphate monohydrate, dibasic sodium phosphate anhydrate, or a combination thereof. In some embodiments, the buffer has a concentration of from about 20 mM to about 30 mM. The pH of the disclosed composition typically is maintained between about 7.0 to about 8.0, with exemplary embodiments having a pH of 7.5. An exemplary tonicity agent is propylene glycol. The tonicity agent may be present in an amount sufficient to achieve a target osmolality of about 230 mOsm/kg to about 320 mOsm/kg. In some embodiments, the target osmolality ranges from about 250 mOsm/kg to about 320 mOsm/kg. To achieve the desired osmolality, some embodiments of the composition comprise about 1% w/v to about 2% w/v of the tonicity agent, with exemplary embodiments comprising 1.6% w/v.

The compositions of the invention comprise at least one of compound I and/or compound II, or a pharmaceutically acceptable salt, solvate, or *N*-oxide thereof. The compositions can further comprise at least one additional pharmacologically active agent. The pharmacologically active agent can be a compound capable of treating, ameliorating, and/or preventing an eye disease or disorder. In some embodiments, the additional pharmacologically active agent can be selected from a corticosteroid, an antihistamine, an antibiotic, an anti-inflammatory, an antiviral, a glaucoma medication, or a combination thereof. Combinations of these pharmacologically active agents also may be used.

One exemplary composition comprises about 7.5% w/v K30-grade polyvinylpyrrolidone, a phosphate buffer having a concentration of about 25 mM and comprising monobasic sodium phosphate monohydrate and dibasic sodium phosphate anhydrate, and 1.6% w/v propylene glycol.

Compositions of the present invention comprise compound I and/or compound II (or a pharmaceutically acceptable salt, solvate, or *N*-oxide thereof).

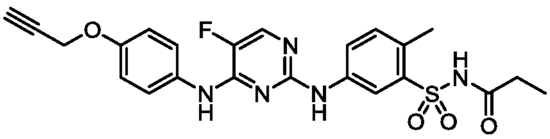

**Compound I**

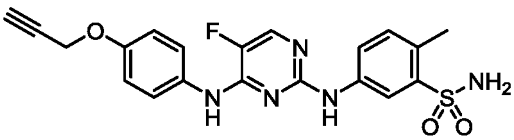

**Compound II**

These composition embodiments may further comprise a solubilizer, a buffer, a tonicity agent, and combinations thereof. An exemplary embodiment of such compositions can comprise from about 2 mg/mL to about 5 mg/mL of compound I (or a pharmaceutically acceptable salt, *N*-oxide, or a solvate thereof), about 1% w/v to about 10% w/v K30-grade polyvinylpyrrolidone, a phosphate buffer having a concentration of about 25 mM, and propylene glycol in an amount sufficient to achieve a target osmolality amount of 300 mOsm/kg.

Also disclosed herein are compositions in which compounds I and/or II may exist in alternate forms, such as prodrugs (for example, compound I can comprise one or more additional progroups), pharmaceutically acceptable salts, *N*-oxides, and hydrates.

In certain embodiments, compounds I and/or II may be formulated as a choline salt. The effective amount of compounds I and/or II (or a pharmaceutically acceptable salt, solvate, or *N*-oxide thereof) may range from about 1 mg/mL to about 25 mg/mL. In certain embodiments, the effective amount of compounds I and/or II (or a pharmaceutically acceptable salt, solvate, or *N*-oxide thereof) ranges from about 2 mg/mL to about 20 mg/mL.

In one embodiment, such compositions of compound I contain up to about 0.5% compound II. In particular disclosed embodiments, the physical and chemical properties of the disclosed compositions are optimized to minimize or prevent precipitation of a compound II even up to 0.5% compound II. Disclosed compositions may, however, comprise this compound in suitably limited amounts, such as an amount less than about 300 $\mu$g/mL.

The disclosed compositions are useful for treating eye diseases or disorders. For example, eye diseases treatable by the disclosed composition include dry eye syndrome, diabetic retinopathy, macular degeneration uveitis, allergic conjunctivitis, glaucoma, rosacea, and combinations thereof. In particular disclosed embodiments, the eye disease or disorder is keratitis sicca. In another embodiment, the disclosed composition is useful to promote hair growth. As such, the composition is useful to treat alopecia and hypotrichosis, including hypotrichosis of the eyebrows and/or eyelashes by increasing their growth.

An exemplary embodiment of the disclosed composition is used to treat keratitis sicca and comprises greater than 2% w/v to about 15% w/v of a solubilizer, a buffer, and a tonicity agent. In other embodiments, the keratitis sicca may be treated using a disclosed composition comprising an effective amount of compounds I and/or II (or a pharmaceutically acceptable salt, solvate, or *N*-oxide thereof), a solubilizer, a buffer, a tonicity agent, or combinations thereof.

Also disclosed herein is a method for administering a composition. In some instances, the method comprises administering a composition comprising greater than 2% w/v to about 15% w/v polyvinylpyrrolidone. Such a method also can comprise administering a composition further comprising a buffer, and a tonicity agent. In other instances, the method concerns administering a composition comprising an effective amount of compounds I and/or II (or a pharmaceutically acceptable salt, solvate, or *N*-oxide thereof), a solubilizer, a buffer, a tonicity agent, or combinations thereof.

In some instances, the method comprises providing an effective amount of a disclosed composition, and applying the disclosed composition to a subject's eye. In particular instances, applying comprises exposing the subject's eye to a disclosed composition via topical placement or injection. Topical placement may involve placing one or more drops of the disclosed composition onto the subject's eye. In yet other instances, the method may further comprise performing one or more non-drug interventions. The one or more non-drug interventions may be selected from punctal occlusion, fitting the subject with scleral or semi-scleral contact lenses that create a fluid-filled layer over the cornea, and combinations thereof. The method may further comprise administering one or more pharmacologically active agents, such as a corticosteroid, an antihistamine, an antibiotic, an anti-inflammatory, an antiviral, a glaucoma medication, or combinations thereof.

The foregoing and other objects, features, and advantages of the invention will become more apparent from the following detailed description, which proceeds with reference to the accompanying figures.

## BRIEF DESCRIPTION OF THE DRAWINGS

FIG. 1 is a graph of solubility (expressed as log S in $\mu$g/mL) versus pH, illustrating the effect of pH on the solubility of compound I choline salt.

FIG. 2 is a graph of observed rate coefficient (as a logarithm) versus pH of a solution of an embodiment of the disclosed composition, which illustrates the pH effects on the conversion rate of compound I choline salt to compound II at various different temperatures.

## DETAILED DESCRIPTION

### I. Introduction

The following term definitions are provided to aid the reader, and should not be considered to provide a definition different from that known by a person of ordinary skill in the art. And, unless otherwise noted, technical terms are used according to conventional usage.

Although methods and materials similar or equivalent to those described herein can be used to practice or test the disclosed technology, suitable methods and materials are described below. The materials, methods, and examples are illustrative only and are not intended to be limiting.

As used herein, the singular terms "a," "an," and "the" include plural referents unless context clearly indicates otherwise. Similarly, the word "or" is intended to include "and" unless the context clearly indicates otherwise. Also, as used herein, the term "comprises" means "includes." Hence "comprising A or B" means including A, B, or A and B.

In order to facilitate review of the various embodiments of the disclosure, the following explanations of specific terms are provided:

**Corticosteroids:** Corticosteroids are steroid hormones that are involved in a wide range of physiologic systems, such as stress response, immune response and regulation of inflammation, carbohydrate metabolism, protein catabolism, blood electrolyte levels, and behavior. Examples of corticosteroids include cortisol, prednisone and prednisilone. Corticosteroids can be administered to a subject by various methods including orally, parenterally (for example by injection) or by direct topical administration in the eye with eye drops. Corticosteroids may be combined with compounds I and/or II (or a pharmaceutically acceptable salt, solvate, or *N*-oxide thereof) in a composition.

**Keratitis sicca (or keratoconjunctivitis sicca, dry eye syndrome):** A dry eye condition associated with decreased tear production or increased evaporation, and having various etiologies, such as inflammatory, non-inflammatory, traumatic, iatrogenic, drug-induced, rosacea-associated, or idiopathic origins.

**Non-steroidal anti-inflammatory drug (NSAID):** A type of anti-inflammatory agent that works by inhibiting the production of prostaglandins. NSAIDS exert anti-inflammatory, analgesic and antipyretic actions. Examples of NSAIDS include ibuprofen, ketoprofen, piroxicam, naproxen, sulindac, aspirin, choline subsalicylate, diflunisal, fenoprofen, indomethacin, meclofenamate, salsalate, tolmetin and magnesium salicylate. These agents can be administered either orally, parenterally (for example by injection) or by direct topical instillation in the eye with eye drops, and they may be combined with compounds I and/or II (or a pharmaceutically acceptable salt, solvate, or *N*-oxide thereof) in a composition.

**Subject:** Refers to humans and non-human subjects, such as mammals.

**Pharmaceutically acceptable salt:** A particular form of a parent compound, wherein the parent compound is modified to include one or more organic and inorganic counter ions.

**Pharmaceutically effective amount (or therapeutically effective amount):** An amount of a compound sufficient to treat, ameliorate, or prevent a specified disorder or disease or one or more of its symptoms and/or to prevent the occurrence of the disease or disorder.

**Restoration of normal tear production:** This phrase refers to the cessation of dry eye symptoms as described in standard ophthalmic practice, such as a response score of less than 14.5 in McMonnies & Ho Dry Eye questionnaire, test results (for example red phenol, fluorescein and the like as known to those skilled in ophthalmological practice) or a combination of indications.

**Significantly increases tear production:** This phrase means a statistically significant (such as $p < 0.05$) increase in tear production as measured by standard ophthalmic practice. For example tear production can be measured by the Schirmer test, the phenol red thread test, tear breakup time (such as by fluorescein staining), Rose Bengal staining, and the like.

## II. Compounds

Disclosed herein are compounds, and compositions thereof, for treating diseases and/or disorders of the eye. In particular disclosed embodiments, such compounds are compound I and/or compound II (or a pharmaceutically acceptable salt, solvate, or *N*-oxide thereof), both of which are suitable for treating one or more eye diseases or disorders. In some embodiments, compounds I and/or II (or a pharmaceutically acceptable salt, solvate, or *N*-oxide thereof) may be capable of inhibiting JAK3 and/or Syk Kinase. In particular disclosed embodiments, compounds I and/or II may exist in particular forms, such as a pharmaceutically acceptable salt, *N*-oxide, or solvate. Compounds used in certain disclosed composition embodiments can be a choline salt, such as a compound I choline salt.

Compound I, having a formula illustrated below, is referred to as 5-fluoro-N2-(4-methyl-3-propionylaminosulfonylphenyl)-N4-[4-(prop-2-ynyloxy)phenyl]-2,4-pyrimidinediamine.

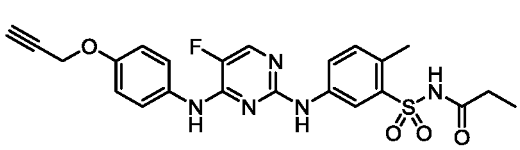

**I**

Compound II, having a formula illustrated below, is referred to as N2-(3-aminosulfonyl-4-methylphenyl)-5-fluoro-N4-[4-(prop-2-ynyloxy)phenyl]-2,4-pyrimidinediamine.

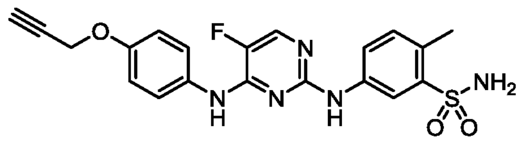

II

Compound I is a prodrug of compound II. Compound I may be pharmacologically active in its prodrug form. In other disclosed embodiments, compound I may be converted into compound II, which is also pharmacologically active. The mechanism by which the propionyl progroup is removed is not critical, and can be caused by, for example, hydrolysis under the acidic conditions of the stomach. Enzymes present in the digestive tract and/or tissues or organs of the body, for example, esterases, amidases, lipolases, phosphatases including ATPases and kinases, cytochrome P450 enzymes found in the liver, and the like, may also remove the propionyl group. Particular embodiments of the composition are used to treat eye diseases or disorders and therefore may be administered directly to the eye. In such embodiments, compound I may be converted to compound II by enzymes found in tears, such as esterases. In some embodiments, administration may include not only topical, but also injection and the like.

One of ordinary skill in the art will appreciate that compounds disclosed herein may exhibit tautomerism, conformational isomerism and/or geometric isomerism. It should be understood that the invention encompasses any tautomeric, conformational isomeric and/or geometric isomeric forms of such compounds, as well as mixtures of these various different isomeric forms. Atropisomers are stereoisomers resulting from hindered rotation about single bonds where the barrier to rotation is high enough to allow for the isolation of the conformers (Eliel, E. L.; Wilen, S. H. Stereochemistry of Organic Compounds; Wiley & Sons: New York, 1994; Chapter 14). Atropisomerism is significant because it introduces an element of chirality in the absence of stereogenic atoms. Disclosed embodiments of the present invention encompass atropisomers, for example in cases of limited rotation about bonds between the 2,4-pyrimidinediamine core structure and groups attached thereto, or for example about bonds between the sulfonamide and the phenyl ring to which it is attached.

The compounds disclosed herein may be present in compositions, or administered to a subject as a salt(s). Such salts include salts suitable for pharmaceutical uses ("pharmaceutically acceptable salts"), salts suitable for veterinary uses, etc. Such salts may be derived from acids or bases, as is well-known in the art.

Any pharmaceutically acceptable salt may be used. Because certain disclosed compounds have both basic groups, for example pyrimidine nitrogen atoms, and acidic groups, for example acidic protons on the sulfonamide and/or the nitrogen atoms at N2 and N4 of the pyrimidinediamine system, these compounds can form pharmaceutically acceptable acid or base-addition salts. Generally, pharmaceutically acceptable salts are those salts that retain substantially one or more of the desired pharmacological activities of the parent compound and which are suitable for administration to humans. Pharmaceutically acceptable salts include acid addition salts formed with inorganic acids or organic acids. Inorganic acids suitable for forming pharmaceutically acceptable acid addition salts include, by way of example and not limitation, hydrohalide acids (for example, hydrochloric acid, hydrobromic acid, hydroiodic acid, etc.), sulfuric acid, nitric acid, phosphoric acid, and the like. Organic acids suitable for forming pharmaceutically acceptable acid addition salts include, by way of example and not limitation, acetic acid, trifluoroacetic acid, propionic acid, hexanoic acid, cyclopentanepropionic acid, glycolic acid, oxalic acid, pyruvic acid, lactic acid, malonic acid, succinic acid, malic acid, maleic acid, fumaric acid, tartaric acid, citric acid, palmitic acid, benzoic acid, 3-(4-hydroxybenzoyl) benzoic acid, cinnamic acid, mandelic acid, alkylsulfonic acids (for example, methanesulfonic acid, ethanesulfonic acid, 1,2-ethane-disulfonic acid, 2-hydroxyethanesulfonic acid, etc.), arylsulfonic acids (for example, benzenesulfonic acid, 4-chlorobenzenesulfonic acid, 2-naphthalenesulfonic acid, 4-toluenesulfonic acid, camphorsulfonic acid, etc.), 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, xinafoate, and the like.

Pharmaceutically acceptable salts also include salts formed when an acidic proton present in the parent compound is either replaced by a metal ion (for example, an alkali metal ion, an alkaline earth metal ion or an aluminum ion) or coordinates with an organic base. Exemplary pharmaceutically acceptable inorganic salts described herein include ammonium salts, sodium salts, potassium salts, calcium salts, and the like. Exemplary salts of the present compounds formed with organic bases include, without limitation, amino acid salts, such as arginine salts and lysine salts; and those formed from other organic bases, including by way of example choline, ethanolamine, diethanolamine, triethanolamine, *N*-methylglucamine, morpholine, piperidine, dimethylamine, diethylamine, triethylamine, and the like.

The compounds disclosed herein also may be administered to a subject as a solvate *(e.g.,* a hydrate), an *N*-oxide, a choline salt, or combinations thereof.

### III. Compositions

Embodiments of the composition disclosed herein effectively treat, ameliorate, and/or prevent one or more eye diseases or disorders, such as, without limitation, dry eye syndrome, diabetic retinopathy, macular degeneration uveitis, allergic conjunctivitis, glaucoma, rosacea, and combinations thereof. Embodiments of the disclosed composition exhibit improved characteristics in comparison to compositions known generally in the art. In some embodiments, the characteristics that are improved include, but are not limited to, physical and/or chemical stability of compounds that may be included in embodiments of the disclosed composition (*e.g.*, compound I and/or compound II, or a pharmaceutically acceptable salt, *N*-oxide, or solvate thereof) and the ability to administer a high concentration of such compounds. In additional embodiments, the ability to maintain desirable physiological properties (*e.g.*, sterile, isotonic, neutral pH, preservative free, and the like) can be achieved using particular embodiments of the disclosed composition.

A composition of the invention comprises polyvinylpyrrolidone. The polyvinylpyrrolidone is present in an amount of from 5% w/v to 15% w/v. Within this range, the maximum amount of the polyvinylpyrrolidone can be any amount such that the polyvinylpyrrolidone remains miscible in water. The maximum amount of the polyvinylpyrrolidone that can be used also may be such that the viscosity of the disclosed composition is suitable for ophthalmic administration. In particular disclosed embodiments, the desired viscosity of the disclosed composition is obtained by varying the amount of the polyvinylpyrrolidone used in the composition and/or by varying the molecular weight of the polyvinylpyrrolidone. For example, low molecular weight polymeric polyvinylpyrrolidones, such as K30-grade polyvinylpyrrolidone, can be used in high amounts. A composition of the invention comprises polyvinylpyrrolidonehaving an average molecular weight ranging from 40,000 to 60,000. In some embodiments, the polyvinylpyrrolidone employed has peroxide levels low enough to ensure adequate stability of the formulation. The polyvinylpyrrolidone contains less than about 400 ppm hydrogen peroxide.

In some embodiments, the composition comprising the polyvinylpyrrolidone can further comprise a buffer and a tonicity agent. The buffer may be a phosphate buffer, such as a buffer comprising monobasic sodium phosphate monohydrate, dibasic sodium phosphate anhydrate, or combinations thereof. The buffer typically has a concentration ranging from about 20 mM to about 30 mM. The buffer typically is selected to maintain a desired pH, such as from about 7.0 to about 8.0. In certain embodiments, the pH is maintained at 7.5 using a sodium phosphate monohydrate buffer and a dibasic sodium phosphate anhydrate buffer having a concentration of 25 mM.

The tonicity agent is selected to provide a desired osmolality. In some embodiments, the osmolality ranges from about 230 mOsm/kg to about 300 mOsm/kg, such as from about 250 mOsm/kg to about 300 mOsm/kg. In exemplary embodiments, the tonicity agent is an alkylene polyol, such as propylene glycol or glycerin. Other suitable tonicity agents include, but are not limited to, metal salts, such as sodium chloride, potassium chloride, and the like; carbohydrates, or sugar alcohols, such as dextrose, mannitol, and the like; and combinations thereof. In some embodiments, the disclosed composition comprises from about 1% w/v to about 2% w/v propylene glycol. Exemplary embodiments use 1.6% propylene glycol.

Disclosed compositions can comprise greater than 2% w/v to about 10% w/v K30-grade polyvinylpyrrolidone, a phosphate buffer at a concentration of about 25 mM (e.g., monobasic sodium phosphate monohydrate and dibasic sodium phosphate anhydrate), and about 1.6% w/v propylene glycol. These compositions typically have a pH ranging from about 7.0 to about 8.0; more typically the pH is 7.5. Exemplary embodiments have an osmolality of about 275 (± 45) mOsm/kg.

A composition of the invention comprises compound I or compound II (or a suitable salt, such as a choline salt, *N*-oxide, or solvate thereof). Such embodiments also can further comprise a solubilizer, a buffer, a tonicity agent, one or more of the other therapeutic compounds, excipients, or combinations thereof.

Compound II has an aqueous solubility of about 0.9 μg/mL at room temperature, which limits the amount of the compound that can be delivered in, for example, an eye drop. Thus, certain disclosed composition embodiments comprise a choline salt of compound I. The choline salt of compound I has significantly higher aqueous solubility than both its free acid form and compound II. Accordingly compound I choline salt can be used to deliver a greater concentration of compound II than can be delivered using compound II directly.

For composition embodiments useful for delivering compound II, a particular feature disclosed herein is formulation of the composition so that the chemical and/or physical properties of the compound(s) are maintained and an effective amount of compound II can be delivered to the eye. For example, compound I (or compound I choline salt) converts to compound II under various conditions. Because compound II has limited solubility relative to compound I (and in particular its choline salt), compound II may precipitate out of solution. This process preferably should be controlled to provide a composition exhibiting little to no precipitation, which requires limiting the amount of compound II present. Accordingly, particular embodiments disclosed herein concern controlling the conversion of compound I (or compound I choline salt) and the precipitation of compound II. For example, solution kinetic studies have been used to determine the amount of compound I choline salt that converts to compound II (FIG. 2). In particular disclosed embodiments, about 5% of compound I choline salt may be converted to compound II in a solution having a pH of 8 and when stored at room

temperature for approximately 16 months. This chemical conversion may lead to precipitation of compound II. Controlling these chemical and/or physical properties to minimize the amount of compound II present in the certain embodiments of the disclosed composition can facilitate making desired compositions; however, the composition need not be completely free of compound II in order to be acceptable.

Also disclosed is a composition wherein the chemical and/or physical properties of compound I and/or compound II are controlled or improved by manipulating the chemical nature of the compound. For instance, compound I is an amphoteric molecule that forms salts with strong bases and therefore may further be used in a salt form. Exemplary compositions include the choline salt of compound I and typically are prepared as a 1:1 choline salt. Typically, the choline salt has higher aqueous solubility than either one of compound I and/or compound II. For example, the choline salt of compound I has a solubility of about 9.1 mg/mL in water.

The choline salt may form a hydrate, such as a monohydrate. Hydrate formation for the choline salt desirably is limited or substantially prevented in order to maintain a high solubility of the choline salt. The monohydrate form of compound I choline salt has an aqueous solubility of about 5 mg/mL at pH 8.8 and room temperature, which is not as high as the non-hydrate form of the compound. Accordingly, one aspect disclosed herein concerns developing a composition wherein a choline salt hydrate, such as a choline salt monohydrate, is limited or substantially prevented.

The concentration of compounds for use in composition embodiments comprising compound I and/or compound II is another consideration. In ophthalmic solutions, it may be desirable to use a therapeutic compound at a concentration that is as high as possible while still maintaining desired performance and/or composition characteristics. In particular disclosed embodiments, the concentration of compound I and/or compound II (or a pharmaceutically acceptable salt, *N*-oxide, or solvate thereof) may range from about 1 mg/mL to about 25 mg/mL; more typically from about 2 mg/mL to about 20 mg/mL, such as 2 mg/mL, 2.5 mg/mL, 3 mg/mL or 5 mg/mL, or from about 5 mg/mL to about 20 mg/mL. In particular disclosed embodiments, the concentration of compound I and/or compound II used in certain embodiments of the disclosed composition may be estimated from data from various studies, such as *in vitro, in vivo,* and *ex vivo* studies. The data may be obtained from animal models, or human models.

Maintaining physiological properties of the disclosed composition embodiments, such as sterility, isotonicity, viscosity, neutrality, purity, and the like, is another factor contemplated. Additional considerations include making compositions sufficiently stable for storage and/or packaging for use. Maintaining desired physiological properties of the disclosed composition may come from using components comprising additional components that can aid in any or more of the following: solubility, nucleation inhibition, compound stability, and/or compatibility.

In particular instances, the disclosed composition may comprise any one or more of the following, in any combination: polyvinylpyrrolidone; one or more solubility enhancers; one or more tonicity agents as disclosed herein; one or more nucleation inhibitors; and/or one or more buffering agents as disclosed herein.

Particular disclosed compositions can comprise one or more solubility enhancers to increase the solubility of compound I and/or compound II in embodiments comprising such compounds. Exemplary solubility enhancers include, but are not limited to, a solubilizer (*e.g.*, propylene glycol monocaprylate, oleoyl macrogol-6 glycerides, oleoyl polyoxyl-6 glycerides, caprylocaproyl macrogol-8 glycerides, caprylocaproyl polyoxyl-8 glycerides, propylene glycol monolaurate, polyglyceryl-3 dioleate, polyvinylpyrrolidone [which can be referred to herein as PVP or povidone], or combinations thereof); a cosolvent (*e.g.*, polyalkylene glycols, such as PEG ethers with one suitable example being PEG 400); and a surfactant (*e.g.*, polysorbate, poloxamer, polyosyl 40 stearate, polyoxyl castor oil, tyloxapol, triton and sorbitan monolaurate, particularly, PS 80, tyloxapol, MYS-40, or combinations thereof).

Various amounts of the solubility enhancers may be used in composition embodiments comprising compound I and/or compound II (or a pharmaceutically acceptable salt, *N*-oxide, or solvate thereof). For example, greater than zero to about 15% (w/w or w/v), or about 1% (w/w or w/v) to about 15% (w/w or w/v) of the solubilizer (*e.g.*, propylene glycol monocaprylate, oleoyl macrogol-6 glycerides, oleoyl polyoxyl-6 glycerides, caprylocaproyl macrogol-8 glycerides, caprylocaproyl polyoxyl-8 glycerides, propylene glycol monolaurate, polyglyceryl-3 dioleate, polyvinylpyrrolidone) may be used, but more typically about 5% (w/w or w/v) to about 10% (w/w or w/v), even more typically about 7.5% (w/w or w/v) to about 10% (w/w or w/v) is used. The cosolvent may be used in an amount ranging from about 0.25% (w/w or w/v) to about 2.5% (w/w or w/v), more typically from about 0.5% (w/w or w/v) to about 2.25% (w/w or w/v), even more typically from about 0.5% (w/w or w/v) to about 2% (w/w or w/v). The amount of the surfactant typically ranges from about 0.2% (w/w or w/v) to about 1.5% (w/w or w/v), more typically from about 0.3% (w/w or w/v) to about 1.25% (w/w or w/v), and even more typically from about 0.5% (w/w or w/v) to about 1% (w/w or w/v).

Embodiments of the disclosed composition also may comprise a tonicity agent. Exemplary tonicity agents useful for forming embodiments of the disclosed composition may be selected from alkylene polyols, such as propylene glycol, glycerin, and the like; metal salts, such as sodium chloride, potassium chloride, and the like; carbohydrates, or sugar alcohols, such as dextrose, mannitol, and the like; and combinations thereof. In particular disclosed embodiments, the tonicity agent is propylene glycol, sodium chloride, or combinations thereof. In particular disclosed embodiments, the amount of the tonicity agent ranges from about 1% (w/w or w/v) to about 2% (w/w or w/v), more typically from about 1.2% (w/w or w/v) to about 1.8% (w/w or w/v), and even more typically from about 1.3% (w/w or w/v) to about 1.7% (w/w

or w/v).

Embodiments of the disclosed composition may further comprise a nucleation inhibitor, such as hydroxypropylmethyl cellulose (HPMC). Nucleation inhibitors are used to preclude, or at least substantially inhibit, nucleation and thus prevent precipitation of compounds I and/or compound II from solution. The amount of nucleation inhibitor used may range from about 0.1% (w/w or w/v) to about 1.5% (w/w or w/v), more typically from about 0.25% (w/w or w/v) to about 1% (w/w or w/v), and even more typically from about 0.25% (w/w or w/v) to about 0.75% (w/w or w/v).

The pH of certain embodiments of the disclosed composition may also be modified to increase solubility and/or maintain maximum stability of compound I and/or compound II. The effects of pH on the solubility of compound I choline salt are provided by FIG. 1. In particular disclosed embodiments the pH is maintained as close to physiological pH (7.4) as possible to avoid eye irritation and discomfort. Embodiments of the disclosed composition may further comprise a buffer (*e.g.*, phosphate buffer, such as monobasic or dibasic sodium phosphate) in order to maintain the desired pH. Typically, the buffer strength is minimized in order to reduce buffer irritation effects. Exemplary components used in disclosed composition studies are provided below in Table 1.

**Table 1: Excipients for Ophthalmic Solution Compositions**

| Excipients | Vender | Grade | Comments |
|---|---|---|---|
| Povidone K-30 | Spectrum | USP or equivalent | Polymer |
| Tyloxapol | Spectrum | N/A | Surfactant |
| Propylene glycol (PG) | Spectrum | USP or equivalent | Cosolvent |
| Edetate Disodium (EDTA) | Spectrum | USP or equivalent | Chelating |
| Monobasic Sodium Phosphate, monohydrate ($NaH_2PO_4 \cdot H_2O$) | Spectrum | USP or equivalent | Buffer |
| Dibasic Sodium Phosphate, anhydrous ($Na_2HPO_4$), | Spectrum | USP or equivalent | Buffer |
| Sodium sulfite $Na_2SO_3$) | Spectrum | FCC or equivalent | Antioxidant |
| Purified Water | | USP or equivalent | |

An exemplary product profile for certain disclosed compositions is provided below in Table 2.

**Table 2: Target product profile for ophthalmic solution**

| Attribute | Target |
|---|---|
| Compound I strength | 0.1 - 0.5% (or the highest achievable) |
| Dosage Form | solution |
| Excipient | commonly used ophthalmic excipients |
| Tonicity | isotonic (290-310 mOsm ) |
| pH | matching pH as closely as possible to tear pH (7-8) |
| Storage | room temperature (15-25 °C) |
| Shelf life | Approximately 2 years |
| Container | LDPE dropper bottles |
| Sterilization | filtration |

In some embodiments, composition embodiments may be tested for chemical and/or physical stability by exposure to elevated temperatures relative to room temperature in order to generate compound II and to evaluate the impact of compound II on physical stability of certain compositions disclosed herein. Physical stability may be evaluated by storing compositions at various temperatures for a certain period of time. For example, embodiments of the disclosed composition may be stored at temperatures ranging from about 2 °C to about 8 °C. Alternatively, compound I and/or compound II may be stored at room temperature. In particular disclosed embodiments, compound I and/or compound II may be stored at any of these temperatures for about 5 weeks.

The physical and/or chemical stability of embodiments of the disclosed composition may be tested by heating the compositions at temperatures above room temperature, such as at temperatures ranging from about 40 °C to about 100 °C; more typically from about 40 °C to about 80 °C. Composition embodiments also may be subject to various temperature cycling conditions to determine composition stability. Particular temperature cycling conditions include freeze/thaw conditions, freezing/heating conditions, cooling/heating conditions, or combinations thereof. Various disclosed composition embodiments were exposed to freeze/thaw conditions (*e.g.*, about - 20 °C to room temperature), freezing/heating conditions (*e.g.*, about -20 °C to about 50 °C), and cooling/heating conditions (*e.g.*, about 5 °C to about

40 °C) using at least one cycle of the particular temperature parameter.

The disclosed compositions may be heated at the temperatures disclosed herein and/or stored at room temperature for a certain period of time in order to determine whether any amount of compound II has formed. In particular disclosed embodiments, the disclosed composition may be heated for about 1 day to about 8 weeks; more typically for about 3 days to about 4 weeks. In particular disclosed embodiments, compound II does not precipitate, and in other embodiments, a minimal amount of precipitation is observed, such as at less than about 7%. Detecting a precipitate may be performed by visual observation or by polarizing light microscopy.

## IV. Synthesis of Compounds I and II and Derivatives Thereof

Compounds I and II, as well as salts III-VII, were synthesized as described below or by analogy to the syntheses described below. Alternative syntheses would be appreciated by one of ordinary skill in the art.

Example 1

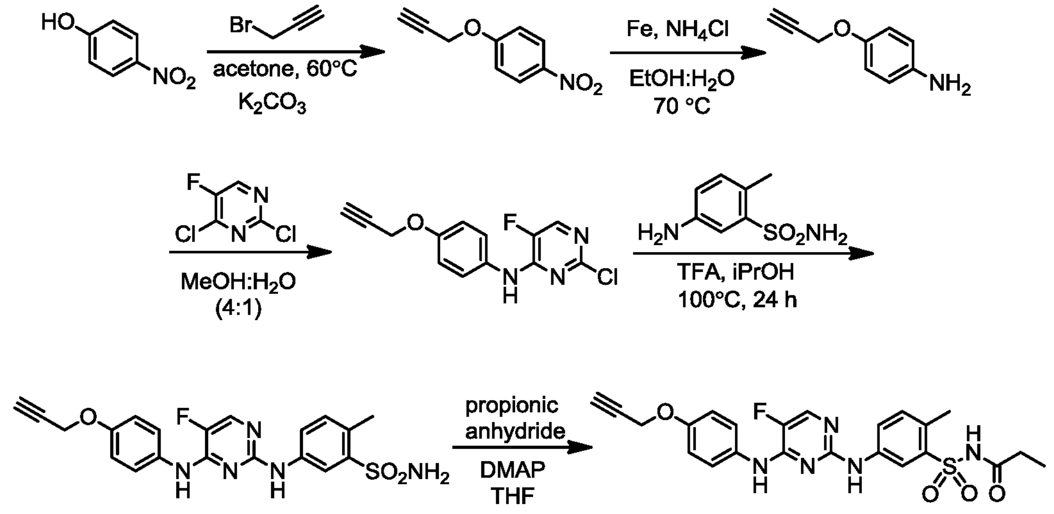


II I

**II: N2-(3-Aminosulfonyl-4-methylphenyl)-5-fluoro-N4-[4-(prop-2-ynyloxy)phenyl]-2,4-pyrimidinediamine**

4-Nitrophenol (1.00 g, 7.19 mmol), propargyl bromide (80 wt % in toluene; 0.788 mL, 7.09 mmol), and $K_2CO_3$ (1.08 g, 7.84 mmol) were combined and stirred in acetone (16.0 mL) at 60°C for 18 hours. The reaction mixture was cooled to room temperature and diluted with water (200 mL). 4-(prop-2-ynyloxy)nitrobenzene was isolated as a white solid by suction filtration (1.12 g). $^1$H NMR ($CDCl_3$): δ 8.22 (d, J= 9.0 Hz, 2H), 7.05 (d, J= 9.0 Hz, 2H), 4.80 (d, J= 2.4 Hz, 2H), 2.59 (t, J= 2.4 Hz, 1H).

4-(Prop-2-ynyloxy)nitrobenzene (0.910 g, 5.13 mmol), iron (1.42 g, 25.3 mmol), and $NH_4Cl$ (0.719g, 12.8 mmol) were vigorously stirred in EtOH/water (1:1, 55 mL) at 70 °C for 15 minutes. The reaction mixture was filtered hot through diatomaceous earth and concentrated *in vacuo.* The residue was suspended in 10% 2N ammoniacal methanol in dichloromethane, sonicated, and filtered through diatomaceous earth. Concentration gave 4-(prop-2-ynyloxy)aniline as an oil which was used without further purification. $^1$H NMR ($CDCl_3$): δ 6.82 (d, J= 8.7 Hz, 2H), 6.64 (d, J= 8.7 Hz, 2H), 4.61 (d, J= 2.4 Hz, 2H), 2.50 (t, J= 2.4 Hz, 1H).

4-(prop-2-ynyloxy)aniline (0.750 g, 5.10 mmol) and 2,4-dichloro-5-fluoropyrimidine (1.27 g, 0.760 mmol, commercially available from Sigma-Aldrich of Milwaukee, Wisconsin, USA) were stirred in MeOH/water (4:1, 35 mL) at room temperature for 18 hours. The reaction mixture was diluted with EtOAc (200 mL) and washed with IN HCl (50 mL) and brine (50 mL). The organic layer was dried ($MgSO_4$), filtered and concentrated *in vacuo.* The residue was purified by column chromatography (silica gel, hexanes ramped to EtOAc:hexanes (1:10)) to provide 2-chloro-5-fluoro-N4-[4-(prop-2-ynyloxy)phenyl]-4-pyrimidineamine as a light brown solid (0.514 g). $^1$H NMR ($CDCl_3$): δ 8.03 (d, J= 2.7 Hz, 1H), 7.53 (d, J= 8.7 Hz, 2H), 7.02 (d, J= 8.7 Hz, 2H), 6.86 (s, 1H), 4.71 (d, J= 2.4 Hz, 2H), 2.55 (t, J= 2.4 Hz, 1H); LCMS: purity: 99%; MS (m/e): 279 ($MH^+$).

2-Chloro-5-fluoro-N4-[4-(prop-2-ynyloxy)phenyl]-4-pyrimidineamine (0.514 g, 1.85 mmol), 3-(aminosulfonyl)-4-methylaniline (0.689 g, 3.70 mmol, made by reduction of commercially available 2-methyl-5-nitrobenzenesulfonamide or synthesized as described below), and trifluoroacetic acid (0.186 mL, 2.41 mmol) were combined with iPrOH (6.0 mL) in a sealed vial and heated at 100°C for 3 hours. The reaction mixture was cooled to room temperature and diluted with IN HCl (80 mL). N2-(3-Aminosulfonyl-4-methylphenyl)-5-fluoro-N4-[4-(prop-2-ynyloxy)phenyl]-2,4-pyrimidinediamine (II) was isolated as a white solid by suction filtration (0.703 g). $^1$H NMR (DMSO-$d_6$): δ 10.08 (bs, 2H), 8.19 (d, J= 4.5 Hz, 1H), 7.89 (s, 1H), 7.74 (dd, J= 2.4 and 8.4 Hz, 1H), 7.58 (d, J= 8.7 Hz, 2H), 7.32 (bs, 2H), 7.23 (d, J= 8.4 Hz, 1H), 6.97 (d, J= 8.4 Hz, 2H), 4.79 (d, J= 2.1 Hz, 2H), 3.59-3.55 (m, 1H), 2.53 (s, 3H); LCMS: purity: 97%; MS (m/e): 428 (MH$^+$).

**I: 5-fluoro-N2-(4-methyl-3-propionylaminosulfonylphenyl)-N4-[4-(prop-2-ynyloxy)phenyl]-2,4-pyrimidinediamine**

N2-(3-Aminosulfonyl-4-methylphenyl)-5- fluoro-N4-[4-(prop-2-ynyloxy)phenyl]-2,4-pyrimidinediamine, **II**, (0.200 g, 0.467 mmol), DMAP (40 mg, 0.33 mmol)) and triethylamine (0.118 mL, 0.847 mmol) were stirred in THF (6.0 mL). Propionic anhydride (0.180 mL, 1.40 mmol) was added to the solution drop wise. The reaction mixture was stirred at room temperature overnight. The solution was diluted with ethyl acetate (50 mL) and washed with water (5 x 25 mL) and brine (10 mL). The organic layer was dried ($MgSO_4$), filtered, and evaporated. The residue was suspended in ethyl acetate (25 mL), sonicated and the solid collected by filtration to give 5-fluoro-N2-(4-methyl-3-propionylaminosulfonylphenyl)-N4-[4-(prop-2-ynyloxy)phenyl]-2,4-pyrimidinediamine, **I**, (0.20 g). $^1$H NMR (DMSO-$d_6$): δ 12.01 (s, 1H), 9.44 (s, 1H), 9.26 (s, 1H), 8.16 (d, J= 2.4 Hz, 1H), 8.06 (dd, J= 0.3 and 3.3 Hz, 1H), 8.00 (dd, J= 2.1 and 7.8 Hz, 1H), 7.69 (d, J= 8.7 Hz, 2H), 7.19 (d, J= 8.4 Hz, 1H), 6.95 (d, J= 8.7 Hz, 2H), 4.77 (d, J= 2.1 Hz, 2H), 3.56 (t, J= 2.1 Hz, 1H), 2.49 (s, 3H), 2.24 (q, J= 7.2 Hz, 2H), 0.89 (t, J= 7.2 Hz, 3H); LCMS: purity: 98%; MS (m/e): 484 (MH$^+$).

**III: 5-fluoro-N2-(4-methyl-3-propionylaminosulfonylphenyl)-N4-[4-(prop-2-ynyloxy)phenyl]-2,4-pyrimidinediamine mono-sodium salt**

5-Fluoro-N2-(4-methyl-3-propionylaminosulfonylphenyl)-N4-[4-(prop-2-ynyloxy)phenyl]-2,4-pyrimidinediamine, **I**, (0.125 g, 0.258 mmol) was suspended in acetonitrile (1.5 mL) and water (1.5 mL) and cooled in an ice bath. A solution of IN NaOH aq. (0.260 mL) was added drop wise. The reaction mixture was stirred until it became clear, filtered through glass wool, and lyophilized to give the sodium salt of **I**. $^1$H NMR (DMSO-$d_6$): δ 9.17 (bs, 2H), 8.01 (d, J= 3.6 Hz, 1H), 7.89 (s, 1H), 7.78-7.69 (m, 3H), 6.99-6.92 (m, 3H), 4.76 (d, J= 2.1 Hz, 1H), 2.43 (s, 3H), 1.95 (q, J= 7.2 Hz, 2H), 0.86 (t, J= 7.2 Hz, 3H); LCMS: purity: 98%; MS (m/e): 484 (MH$^+$).

The following compounds were made in a similar fashion to those above.

**IV: 5-Fluoro-N2-[4-methyl-3-(N-propionylaminosulfonyl)phenyl]-N4-[4-(2-propynyloxy)phenyl]-2,4-pyrimidinediamine Potassium Salt**

$^1$H NMR (DMSO-$d_6$): δ 9.16 (s, 1H), 9.14 (s, 1H), 8.01 (d, J= 3.6 Hz, 1H), 7.85 (d, J= 2.1 Hz, 1H), 7.75-7.70 (m, 3H), 6.97-6.92 (m, 3H), 4.76 (d, J= 1.8 Hz, 2H), 3.55 (t, J= 2.4 Hz, 1H), 2.42 (s, 3H), 1.91 (q, J= 7.5 Hz, 2H), 0.85 (t, J= 7.5 Hz, 3H); LCMS: purity: 97%; MS (m/z): 484 (parent, MH$^+$).

**V: 5-Fluoro-N2-[4-methyl-3-(N-propionylaminosulfonyl)phenyl]-N4-[4-(2-propynyloxy)phenyl]-2,4-pyrimidinediamine Calcium Salt**

$^1$H NMR (DMSO-$d_6$): δ 9.16 (s, 2H), 8.00 (d, J= 3.6 Hz, 1H), 7.88 (d, J= 1.8 Hz, 1H), 7.75-7.69 (m, 3H), 6.97-6.92 (m, 3H), 4.76 (d, J= 1.8 Hz, 2H), 3.55 (t, J= 2.1 Hz, 1H), 2.43 (s, 3H), 1.94 (q, J= 7.5 Hz, 2H), 0.87 (t, J= 7.5 Hz, 3H); LCMS: purity: 98%; MS (m/z): 484 (parent, MH$^+$).

**VI: 5-Fluoro-N2-[4-methyl-3-(N-propionylaminosulfonyl)phenyl]-N4-[4-(2-propynyloxy)phenyl]-2,4-pyrimidinediamine Arginine Salt**

$^1$H NMR ($D_2O$): δ 7.61 (d, J= 3.9 Hz, 1H), 7.57-7.55 (m, 1H), 7.36-7.31 (m, 1H), 7.12 (d, J= 8.7 Hz, 2H), 6.88 (d, J= 8.7 Hz, 1H), 6.72 (d, J= 9.0 Hz, 2H), 4.77-4.75 (m, 2H), 3.60 (t, J= 6.0 Hz, 1H), 3.09 (t, J= 6.9 Hz, 2H), 2.84-2.81 (m, 1H), 2.35 (s, 3H), 2.03 (q, J= 5.7 Hz, 2H), 1.80-1.72 (m, 2H), 1.61-1.48 (m, 2H), 0.855 (t, J= 7.5 Hz, 3H); LCMS: purity: 98%; MS (m/z): 484 (parent, MH$^+$).

**VII: 5-Fluoro-N2-[4-methyl-3-(N-propionylaminosulfonyl)phenyl]-N4-[4-(2-propynyloxy)phenyl]-2,4-pyrimidine-diamine Choline Salt**

$^1$H NMR (DMSO-$d_6$): δ 9.16 (s, 2H), 8.00 (d, J= 3.6 Hz, 1H), 7.85 (d, J= 1.8 Hz, 1H), 7.75-7.69 (m, 3H), 6.97-6.90 (m, 3H), 5.27 (t, J= 4.8 Hz, 1H), 4.76 (d, J= 1.8 Hz, 2H), 3.86-3.77 (m, 2H), 3.56-3.54 (m, 1H), 3.40-3.54 (m, 2H), 3.08 (s, 9H), 2.42 (s, 3H); LCMS: purity: 99%; MS (m/z): 484 (parent, $MH^+$).

**Example 2**

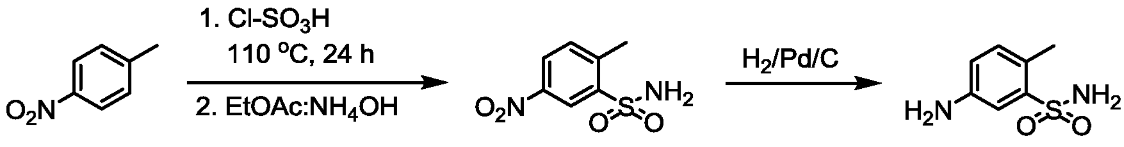


**5-amino-2-methylbenzenesulfonamide**

4-methylnitrobenzene (20 mmol) was treated at 0 °C with chlorosulfonic acid (5.29 mL, 80 mmol) and then, after bringing the homogeneous solution to room temperature, the solution was stirred at 110 °C for 24 hours. The resulting slurry was then poured over ice water (100 gm), extracted with diethyl ether (3 x 75 mL), and the organic phase washed with water (75 mL), then dried over anhydrous sodium sulfate. The solvent was then removed under reduced pressure to afford the crude sulfonyl chloride which was taken up in ethyl acetate and stirred with ammonium hydroxide overnight at room temperature. After the ethyl acetate layer was separated, the aqueous layer was extracted with ethyl acetate. The organic layers were combined, dried over anhydrous sodium sulfate and the solvent was removed under reduced pressure. The oil obtained was purified by column chromatography (silica gel, hexanes then 10%, 20%, up to 50% ethyl acetate in hexanes to afford 3-aminosulfonyl-4-methylnitrobenzene, LCMS: purity: 95 %; MS (m/e): 217 ($MH^+$).

To a solution of 3-aminosulfonyl-4-methylnitrobenzene in dichloromethane and methanol was added 10 % Pd/C and the mixture shaken under a hydrogen atmosphere at 50 psi for 15 minutes. The mixture was filtered through diatomaceous earth and the filter cake was washed with methanol. The combined organic solvents were concentrated under reduced pressure to give crude product, which was further purified by flash column chromatography (ethyl acetate: hexanes 1:1) to give 3-aminosulfonyl-4-methylaniline, LCMS: purity: 87%; MS (m/e): 187 ($MH^+$).

**V. Method of Making Embodiments of the Disclosed Composition**

Disclosed compositions may be made by combining disclosed components in appropriate amounts. Particular disclosed embodiments concern compositions comprising compound I or a compound I choline salt. In some disclosed embodiments, composition embodiments may also comprise a limited amount of compound II (*e.g.,* less than about 300 μg/mL). Suitable composition embodiments also include compositions that do not require compound I, a compound I choline salt, or compound II. In these embodiments, the composition can comprise polyvinylpyrrolidone. Some embodiments can further comprise a buffer and a tonicity agent. Exemplary compositions comprise polyvinylpyrrolidone, a phosphate buffer, and propylene glycol.

Disclosed composition embodiments comprising compound I and/or compound II may be made by preparing two separate solutions, such as a vehicle solution and a solution comprising compound I and/or compound II. The vehicle can be made by weighing a target amount of each component of the composition (other than compound I) into a container sequentially and then adding a solvent, typically water. Typically, an amount less than the target amount of solvent is added in order to mix components and ensure solubility, such as about 2/3 of the target amount. The pH may be adjusted using an appropriate solution, such as a NaOH solution (in order to make the vehicle more basic) or an HCl solution (in order to make the vehicle more acidic). Typically, the pH is adjusted to 7.5 +/- 0.1 or to from about 7.6 to about 7.8. The remaining amount of solvent may be added subsequent to stirring and pH adjustment in order to achieve a target final weight. In particular disclosed embodiment, the density of the vehicle is about 1.02 g/mL.

After making the vehicle, the solution comprising compound I and/or compound II may be added to the vehicle slowly while stirring the solution. If compound I and/or compound II is not entirely dissolved with stirring, then sonication may be used to facilitate dissolution. The pH may be adjusted using the method disclosed above in order to achieve a pH of 7.5 +/- 0.1. Embodiments that do not comprise compound I and/or compound II may be used without further manipulation.

Composition osmolality may be adjusted in order to achieve a target osmolality. For example, the osmolality

may be adjusted to be within a target range of the lacrimal fluid osmolality of 308 mOsm/kg. The osmolality of embodiments of the disclosed composition may be adjusted by adding an amount of NaCl or propylene glycol. The amount of NaCl or propylene glycol to be added may be determined by using Formula I, below, to determine the osmolality of each component used in the disclosed composition and then subtracting the osmolality of each component from a target osmolality (*e.g.*, 300 mOsm/kg or 275 ($\pm$ 45) mOsm/kg).

$$Osmolality\ of\ Ingredient = Qty\ in\ g/L\ x\ (i\ value)\ x1000/\ mol.\ wt = mOsml/Kg \qquad (I)$$

Disclosed compositions may be tested for chemical and/or physical stability by stressing a sample. Samples used for this type of testing can be prepared by sterilizing the disclosed composition, such as by passing the disclosed composition through a micron filter (*e.g.*, 0.2 micron filter) and then transferring an amount of the sterilized composition to a glass ampule, which is then sealed. The samples present in the ampules are then stressed at various temperatures. The samples are analyzed after stressing at different time points in order to analyze, for example, appearance and impurity profiles.

Various excipients disclosed herein can be added to form embodiments of the disclosed composition. These excipients can be added as individual components, mixtures, solutions, and or suspensions. Table 3 provides examples of excipients useful for forming topical ophthalmic compositions disclosed herein.

**Table 3: Exemplary excipients for use in the present ophthalmic compositions**

| Excipients | Function | Concentrations |
|---|---|---|
| PVP K30 | Solubilizer and/or viscosity enhancer | 1-15% |
| HPMC E5 | Precipitation inhibitor and/or viscosity enhancer | 0.45-1.0% |
| Polysorbate 80 | Surfactant | 0.1-4% |
| Tyloxapol | Surfactant | 0.3% |
| MYS-40 | Surfactant | 1% |
| PEG 400 | Cosolvent | 1% |
| PG/Sodium Chloride | Isotonic agent | q.s. to target osmolality (300 mOsm/kg) |
| HCl/NaOH | pH adjusting agent | q.s. to target pH (7-8) |
| Monobasic Sodium phosphate | Buffer | q.s. to target buffer capacity |

**VI. Method of Using the Disclosed Composition**

Disclosed composition embodiments may be used to treat, ameliorate, or prevent one or more eye diseases or disorders. In some embodiments, a composition comprising polyvinylpyrrolidone may be used to treat such diseases or disorders. Such compositions may further comprise a buffer and a tonicity agent. In some embodiments, a composition comprising compound I and/or compound II (or a pharmaceutically acceptable salt, *N*-oxide, or solvate thereof) may be used to treat such diseases or disorders. In these embodiments, the ability of compounds I and/or II (or a pharmaceutically acceptable salt, *N*-oxide, or solvate thereof) to treat, ameliorate, and/or prevent the eye diseases or disorders disclosed herein may be attributed to such compounds' ability to act as potent and selective inhibitor of JAK kinases, and particularly selective for cytokine signaling pathways containing JAK3. Certain embodiments of the disclosed composition may be used in a variety of *in vitro, in vivo* and *ex vivo* contexts to regulate or inhibit JAK kinase activity, signaling cascades in which JAK kinases play a role, and the biological responses effected by such signaling cascades. For example, embodiments of the disclosed composition may be used to inhibit JAK kinase, either *in vitro* or *in vivo,* in virtually any cell type expressing the JAK kinase (*e.g.*, hematopoietic cells in which JAK3 is predominantly expressed). Certain composition disclosed herein may also be used to regulate signal transduction cascades in which JAK kinases, particularly JAK3, play a role. Such JAK-dependent signal transduction cascades include, but are not limited to, the signaling cascades of cytokine receptors that involve the common gamma chain, such as, for example, the IL-4, IL-7, IL-5, IL-9, IL-15 and IL-21, or IL-2, IL-4, IL-7, IL-9, IL-15 and IL-21 receptor signaling cascades. Compounds I and/or II (or a pharmaceutically acceptable salt, solvate, or *N*-oxide thereof) may also be used *in vitro* or *in vivo* to regulate, and in particular inhibit, cellular or biological responses affected by such JAK-dependent signal transduction cascades. Such cellular or biological responses include, but are not limited to, IL-4/ramos CD23 upregulation, IL-2 mediated T-cell proliferation, etc. Importantly, compounds I and/or II (or a pharmaceutically acceptable salt, solvate, or *N*-oxide thereof) may be used to inhibit JAK kinases *in vivo* as a therapeutic approach towards the treatment or prevention of diseases mediated, either wholly or in part, by a JAK kinase activity. Such diseases are referred to as "JAK kinase mediated diseases."

Without being limited to a particular theory of operation, it is currently believed that treatment, amelioration, and/or prevention of eye disorders is due, at least in part, to the components of the compositions disclosed herein. In some embodiments, the eye disease or disorder may be mediated, at least in part, by JAK kinases, and therefore may be treated using composition embodiments comprising compound I and/or compound II. In some embodiments, the eye disease or disorder that can be treated or prevented using embodiments of the disclosed composition include diseases and disorders of the eye including, but not limited to, dry eye syndrome, uveitis, allergic conjunctivitis, glaucoma, sympathetic ophthalmia and rosacea (of the eye).

Dry eye syndrome (DES), otherwise known as keratoconjunctivitis sicca (KCS), keratitis sicca, sicca syndrome, or xerophthalmia, is an eye disease caused by decreased tear production or increased tear film evaporation commonly found in humans and some animals. Uveitis or iridocyclitis refers to inflammation of the middle layer of the eye (the "uvea") and in common usage may refer to any inflammatory process involving the interior of the eye. Allergic conjunctivitis is inflammation of the conjunctiva (the membrane covering the white part of the eye) due to allergy. Glaucoma refers to a group of diseases that affect the optic nerve and involves a loss of retinal ganglion cells in a characteristic pattern, *i.e*., a type of optic neuropathy. Raised intraocular pressure is a significant risk factor for developing glaucoma (above 22 mmHg or 2.9 kPa), and inflammatory processes, e.g., uveitis, can cause this rise in intraocular pressure. Rosacea is a chronic inflammatory condition characterized by facial erythema but it can affect the eyes and nose (rhinophyma). Disclosed composition embodiments may be used to treat such diseases and/or disorders of the eye. In one instance, the disease and/or disorder of the eye is selected from dry eye syndrome, diabetic retinopathy, macular degeneration uveitis, allergic conjunctivitis, glaucoma, rosacea, and combinations thereof. In one instance, the disease and/or disorder of the eye is dry eye syndrome. In another instance, the disease and/or disorder of the eye is uveitis. In one instance, the disease and/or disorder of the eye is allergic conjunctivitis. In one instance, disease and/or disorder of the eye is glaucoma. In another instance, the disease and/or disorder of the eye is rosacea.

Disclosed herein is a method for administering the disclosed composition. The method may comprise providing an effective amount of the composition, and applying the composition to a subject's eye. Any of the disclosed embodiments of the composition may be used. In certain instances, applying the composition comprises exposing the subject's eye to the composition via topical placement or injection. Topical placement typically concerns placing one or more drops of the composition onto the subject's eye.

Also disclosed herein is a method for treating an eye disease or disorder, comprising administering an embodiment of the disclosed composition. The method may further comprise performing one or more non-drug interventions. In particular instances, the one or more non-drug interventions may be selected from punctal occlusion, fitting the subject with scleral or semi-scleral contact lenses that create a fluid-filled layer over the cornea, and combinations thereof. The disclosed method also may further comprise administering one or more pharmacologically active agents, which may be selected from a corticosteroid, an antihistamine, an antibiotic, an anti-inflammatory, an antiviral, a glaucoma medication, and combinations thereof.

Kits are disclosed comprising the any one of the composition embodiments disclosed herein and a container housing the composition, which may be equipped with a separable or integrated dropper.

Embodiments of the disclosed composition may be administered alone, or they may be administered in combination with other agents useful for treating diseases and/or disorders of the eye. The disclosed composition may be administered in combination with agents useful to treat other disorders or maladies, such as steroids, membrane stabilizers, 5-lipoxygenase (5LO) inhibitors, leukotriene synthesis and receptor inhibitors, inhibitors of IgE isotype switching or IgE synthesis, IgG isotype switching or IgG synthesis, β-agonists, tryptase inhibitors, aspirin, cyclooxygenase (COX) inhibitors, methotrexate, anti-TNF drugs, rituxan, PD4 inhibitors, p38 inhibitors, PDE4 inhibitors, and antihistamines, to name a few.

Particular immunosuppressive therapies that can be used in combination with administering a disclosed composition, include for example, mercaptopurine; corticosteroids, such as prednisone, methylprednisolone and prednisolone; alkylating agents, such as cyclophosphamide; calcineurin inhibitors, such as cyclosporine, sirolimus and tacrolimus; inhibitors of inosine monophosphate dehydrogenase (IMPDH), such as mycophenolate, mycophenolate mofetil and azathioprine; and agents designed to suppress cellular immunity while leaving the recipient's humoral immunologic response intact, including various antibodies (for example, antilymphocyte globulin (ALG), antithymocyte globulin (ATG), monoclonal anti-T-cell antibodies (OKT3)) and irradiation. These various agents can be used in accordance with their standard or common dosages, as specified in the prescribing information accompanying commercially available forms of the drugs (see also, the prescribing information in the 2006 Edition of The Physician's Desk Reference).

Azathioprine is currently available from Salix Pharmaceuticals, Inc. under the brand name AZASAN; mercaptopurine is currently available from Gate Pharmaceuticals, Inc. under the brand name PURINETHOL; prednisone and prednisolone are currently available from Roxane Laboratories, Inc.; Methyl prednisolone is currently available from

Pfizer; sirolimus (rapamycin) is currently available from Pfizer under the brand name RAPAMUNE; tacrolimus is currently available from Fujisawa under the brand name PROGRAF; cyclosporine is current available from Novartis under the brand dame SANDIMMUNE and Abbott under the brand name GENGRAF; IMPDH inhibitors such as mycophenolate mofetil and mycophenolic acid are currently available from Roche under the brand name CELLCEPT and Novartis under the brand name MYFORTIC; azathioprine is currently available from Glaxo Smith Kline under the brand name IMURAN; and antibodies are currently available from Ortho Biotech under the brand name ORTHOCLONE, Novartis under the brand name SIMULECT (basiliximab) and Roche under the brand name ZENAPAX (daclizumab).

In one embodiment, embodiments of the disclosed composition can be administered either in combination or adjunctively with an antihistamine, an antibiotic, an anti-inflammatory, an antiviral and a glaucoma medication. Common antihistamines include livostin, patanol, cromolyn, alomide. There are also non-prescription antihistamines for the eye, which are less potent but can be very helpful in milder case, such as pheniramine. Examples of common antibiotics used in the eye are sulfacetamide, erythromycin, gentamicin, tobramycin, ciprofloxacin and ofloxacin. Corticosteroids (sometimes referred to as "steroids") are similar to a natural substance produced by the adrenal gland and are very effective anti-inflammatories for a wide variety of eye problems. Corticosteroids can be safely used in the eye, and do not carry most of the risks associated with oral steroids like prednisone. Corticosteroids used to treat the eye include, but are not limited to, prednisolone, fluorometholone and dexamethasone. Non-steroidal anti-inflammatories for the eye include, but are not limited to, ibuprofen, diclofenac, ketorolac and flurbiprofen. Common antiviral eye medications include, but are not limited to, triflurthymidine, adenine, arabinoside and idoxuridine. Glaucoma medications typically attempt to reduce the eye's intraocular pressure, the fluid pressure inside the eye, to prevent damage to the optic nerve resulting in loss of vision. These medications may lower pressure by decreasing the amount of fluid produced in the eye, by increasing the amount of fluid exiting through the eye's natural drain, or by providing additional pathways for fluid to leave the eye. Often more than one glaucoma medication will be used simultaneously, as these effects can combine to lower pressure even further than possible with one medicine alone. Common glaucoma medications include, but are not limited to, betablockers such as timolol, metipranolol, carteolol, betaxolol and levobunolol; prostaglandin analogues such as latanoprost; cholinergic agonists such as pilocarpine and carbachol; alpha agonists such as bromonidine and iopidine; carbonic anhydrase inhibitors such as dorzolamide; and adenergic agonists such as epinephrine and dipivefrin.

Subjects to be treated with embodiments of the disclosed composition are selected based on a clinical presentation or ophthalmic examination that suggests the presence of diseases and/or disorders of the eye. For example, the subject may complain of an uncomfortable or burning sensation of the eyes. Photophobia or blurred vision may even be present in severe cases. The medical history of the patient may also be suggestive of dry eyes, for example in a patient with a pre-existing diagnosis of acne rosacea, radiation therapy, rheumatoid arthritis, systemic lupus erythematosus, or scleroderma, or other autoimmune disorder. Biomicroscopic examination with a slit lamp is typically performed to detect meibomitis, conjunctival dilation, decreased tear meniscus, increased tear debris, mucus strands or staining patterns consistent with keratoconjunctivitis sicca. A tear breakup time of less than 10 seconds may also be assessed, and the Schirmer test is frequently performed to more objectively identify subjects who would benefit from treatment with the claimed agents. Particular groups of patients may be selected for treatment, for example those who have decreased tear production from the lacrimal glands (for example, those who have a Schirmer's test that suggests hypofunction of the lacrimal gland due to immune-mediated or other disorders).

The disclosed composition may be instilled in the eye using eye drops as frequently as is needed to treat, ameliorate, and/or prevent the eye disease. For example, the disclosed composition may be administered two times, three times, four times, or more per day. Treatment may be continued for at least a week, month, or year, and in some subjects treatment may extend over multiple years.

In particular cases, subjects are selected for concomitant treatment with pharmaceutical, non-pharmaceutical interventions, or combinations thereof. For example, punctal occlusion is performed to decrease the outflow of tears from the eye while the claimed composition increases lacrimal gland tear production. Yet another example is fitting the subject with scleral or semi-scleral contact lenses that create a fluid-filled layer over the cornea.

Combination therapies are also described that combine embodiments of the disclosed composition with another agent that treats another condition, such as a condition associated with the dry eyes. In some examples, the subject is diagnosed with an underlying disorder associated with the dry eyes and the combination therapy is administered to the subject. In one example, the subject is found to have a meibomitis that would be responsive to topical application of corticosteroids, such as a prednisolone acetate ophthalmic suspension 1%. Components of the disclosed composition can be suspended in the prednisolone composition and instilled in the eye 2 to 4 times a day. In other examples, the dry eyes are associated with seasonal allergies or other inflammatory conditions, and the eye drops are administered with or in a composition that includes antihistamines (such as pheniramine, emedastine, or azelastine), decongestants (such as tetrahydrozoline hydrochloride or naphazoline), or a non-steroidal anti-inflammatory agent (such as nepafenac or ketorolac), corticosteroids (such as fluorometholone or loteprednol), mast cell stabilizers (such as azelastie, cromal, emedastine, ketotifen, lodoxamine, nedocromil, olopatadine, or pemirolast). If the dry eyes are associated with an infectious bacterial condition (such a meibomian gland infection or corneal infection) the eye drops are administered with or in a combination composition can contain appropriate antibiotics (such as ciprofloxacin, erythromycin, gentamicin, ofloxacin, sulfacetamine, tobramycin, or monofloxacin). If the dry eyes are associated with a viral infection, the eye drops are administered with or in a combination composition with an anti-viral agent such as trifluridine or idoxuridine.

Another example of a combination therapy is a subject who is diagnosed with ocular rosacea after presenting with irritated eyes and facial erythema with telangiectasia. The subject is treated with eye drops comprising an embodiment of the disclosed composition, and the subject is also treated with an oral antibiotic, such as a tetracycline antibiotic, such as minocycline.

In another example, the subject presents with dry eyes and another pre-existing autoimmune disorder, and is treated with the eye drops comprising the disclosed composition. The subject is also treated with systemic (for example) oral corticosteroid therapy, such as a tapering dose of prednisolone.

In one aspect, treatment results in an improvement of the state of the subject suffering from the dry eye syndrome. This improvement may be demonstrated by one or more of a reduction of inferior corneal fluorescein staining, improvement in a Schirmer test, or improvement in signs and/or symptoms of dry eye syndrome. Such improvement may be measured, by way of example, using the Ocular Surface Disease Index, the Ocular Comfort Index, or the like.

**VII. Working Examples**

Examples with 2% w/w PVP falling outside the scope of the claims are included for reference purposes.

**General Materials:**

- Compound I choline salt
- Povidone (PVP or polyvinylpyrrolidone) K30, USP/EP
- Propylene Glycol (PG), USP/EP
- Monobasic Sodium Phosphate, monohydrate($NaH_2PO_4 \cdot H_2O$), USP/EP
- Dibasic Sodium Phosphate, anhydrous ($Na_2HPO_4$), USP/EP
- Purified Water, USP
- 10N Sodium Hydroxide (NaOH) and 10N Hydrochloric acid (HCl)
- Sterile Polyethersulfone (PES) membrane filters (0.2 $\mu$m pore size)

**Equipment:** Balance, magnetic stirrer or mechanical mixer, glass or stainless steel containers, pH meter, osmometer and miscellaneous lab supplies.

**Vehicle Preparation:** Approximately 2/3 of the required amount of water was transferred into a suitable container and stirred at room temperature. The required amount of PVP was added in small portions to the water while mixing. Mixing was continued at room temperature until a clear solution was achieved. The required amount of $NaH_2PO_4 \cdot H_2O$ and $Na_2HPO_4$ was added into the container and mixed at room temperature until a clear solution was achieved. Water was added to about 95% of the target weight and mixed well. The pH may be adjusted to 7.5 +/- 0.1 with NaOH or HCl. PG (about 1.75%) was added to achieve osmolality of 300 +/- 10 mOsm/kg. A sufficient quantity of water (QS) was added to achieve the target weight (*e.g.,* the density of the formulation is 1.02 g/mL, and the target weight of 100 mL of formulation is 102 g) and mixed thoroughly. The vehicle was filtered with a 0.2 $\mu$m PES filter.

**Exemplary Preparation Methods for Compositions Comprising Compounds I and/or II:**

Formulations were prepared under $N_2$ protection. Approximately 2/3 of the required amount of water was transferred into a suitable container and stirred at room temperature. The required amount of PVP was added in small portions to the water while mixing. Mixing was continued at room temperature until a clear solution was achieved. The required amount of $NaH_2PO_4 \cdot H_2O$ and $Na_2HPO_4$ was added into the container, and mixed at room temperature until a clear solution was achieved. Water was added to about 95% of the target weight and mixed well. The required amount of compound I choline salt was added slowly into the container while stirring. The stirring speed may be adjusted to high and stirring continued at room temperature until a clear solution is achieved (approximately 30 minutes). The pH was measured and can be adjusted to 7.5 +/- 0.1 with NaOH or HCl if needed. If a precipitate forms, stirring should be continued until all precipitate dissolves and a clear solution is achieved. PG (about 1.6 % (w/v) of PG is needed to achieve the target osmolality) was added to achieve osmolality of 300 +/- 10 mOsm/kg. A sufficient quantity of water (QS) was added to achieve the target weight (as above) and mixed thoroughly. The formulation was then filtered with a 0.2 $\mu$m PES filter.

**Chromatographic Analysis**

RAM-1038 is used for compound I choline salt quantitation. The HPLC conditions are summarized as follows:

| | |
|---|---|
| Mobile Phase A: | 0.05% TFA in Water |
| Mobile Phase B: | 0.05% TFA in Acetonitrile |
| HPLC Column: | Waters SymmetryShield™ RP18, 3.5 μm, 4.6 X 150 mm |
| Column Temp.: | 30°C |
| Detection: | 260 nm |
| Run time: | 45 minutes |
| Flow rate: | 1.0 mL/min |
| Injection volume: | 20 μL |
| Diluent: | 40% Acetonitrile - 60% water |

Gradient Profile:

| Time (min) | % A | % B |
|---|---|---|
| 0 | 80 | 20 |
| 20 | 70 | 30 |
| 35 | 48 | 52 |
| 35.5 | 10 | 90 |
| 39.5 | 10 | 90 |
| 40 | 80 | 20 |
| 45 | 80 | 20 |
| *Retention time of compound I choline salt is approximately 19 minutes. | | |

Particular disclosed composition embodiments were tested for compliance with the desired characteristics as disclosed herein. The exemplary embodiments discussed in this section concern compositions comprising the choline salt of compound I; however, the disclosed compositions also may be made using any other compounds disclosed herein or may be made without such compounds (*e.g.*, without compound I and/or II, or pharmaceutically acceptable salts, *N*-oxides, or solvates thereof).

In one embodiment, a composition was made using 10 mM of compound I (concentration calculated based on compound I, not compound I choline salt) in 10 mM phosphate buffer. Varying amounts of PVP K30, PEG 400, and various surfactants were added to multiple different solutions of compound I. Additionally, propylene glycol and/or NaCl were used as tonicity agents in order to adjust the osmolality of the various embodiments of the disclosed composition. A full description of the various embodiments and their components used in this particular embodiment is provided in Table 4.

**Table 4: Prepared compositions for evaluation**

| F# | Compound I eq, mM | PVP K30 %w/w | PS80 %w/w | MYS40 %w/w | Tylox %/w/w | PEG400 %w/w | PG, %w/w | Phosphate buffer, 10mM |
|---|---|---|---|---|---|---|---|---|
| 1 | 10 | 2 | 1 | | | 1 | 1.67 | qs |
| 2 | 10 | 2 | 1 | | | 0.5 | 1.76 | qs |
| 3 | 10 | 2 | | 1 | | 1 | 1.69 | qs |
| 4 | 10 | 2 | | | 1 | 1 | 1.71 | qs |
| 5 | 10 | 2 | 0.5 | | | 1 | 1.70 | qs |
| 6 | 10 | 5 | 0.5 | | | 1 | 1.69 | qs |
| 7 | 10 | 5 | 1 | | | 1 | 1.66 | qs |
| 8 | 10 | 5 | | 1 | | 1 | 1.68 | qs |
| 9 | 10 | 5 | | | 1 | 1 | 1.70 | qs |

(continued)

| F# | Compound I eq, mM | PVP K30 %w/w | PS80 %w/w | MYS40 %w/w | Tylox %/w/w | PEG400 %w/w | PG, %w/w | Phosphate buffer, 10mM |
|---|---|---|---|---|---|---|---|---|
| 10 | 10 | 10 | 0.5 | | | 1 | 1.68 | qs |
| 11 | 10 | 10 | 1 | | | 1 | 1.65 | qs |
| 12 | 10 | 10 | | 1 | | 1 | 1.67 | qs |
| 13 | 10 | 10 | | | 1 | 1 | 1.69 | qs |

All samples of the composition in this particular embodiment were stored for approximately 5 weeks at either about 2 °C to about 8 °C, or at room temperature. Under these conditions, embodiments of the composition comprising 10% PVP and 1% of a surfactant (Samples 11-13) remained as a clear pale yellow solution. These results are provided in Table 5.

**Table 5: Physical appearances of stressed and unstressed compositions**

| F# | Appearance of unstressed compositions stored at RT for 27 days | Appearance of unstressed compositions stored at 2-8°C for 5 wks | Appearance of stressed compositions stored at RT for 4.5 wks |
|---|---|---|---|
| 1 | PPT (after 2.5d) | PPT (after 5d) | N/A |
| 2 | PPT (after 2.5d) | Small amount PPT (after 2.5d) | N/A |
| 3 | PPT (after 1 d) | PPT (after 2.5d) | N/A |
| 4 | PPT (after 2.5d) | PPT | N/A |
| 5 | PPT | PPT | N/A |
| 6 | PPT | PPT | N/A |
| 7 | PPT | PPT | N/A |
| 8 | PPT | clear pale yellow soln | N/A |
| 9 | Less PPT | clear pale yellow soln | N/A |
| 10 | A few PPT | clear pale yellow soln | PPT |
| 11 | clear pale yellow soln | clear pale yellow soln | clear pale yellow soln |
| 12 | clear pale yellow soln | clear pale yellow soln | clear pale yellow soln |
| 13 | clear pale yellow soln | clear pale yellow soln | clear pale yellow soln |
| *PPT: precipitation observed | | | |

Also in this embodiment, various different composition embodiments were stressed at 80 °C for up to three days. In order to determine the level of precipitation of compound II, if any, these composition embodiments were analyzed using HPLC before and after stressing. Embodiments of this exemplary composition comprising 2% PVP, either MYS40 or PS80, and 1% PEG400 did not provide adequate stability, as these samples exhibited precipitation after stressing. The results of other embodiments of the disclosed composition are provided in Table 6, below.

**Table 6: Analytical results of particular compositions**

| Samples | Conditions | Appearance | pH | Purity % | %LS | Compound II %area |
|---|---|---|---|---|---|---|
| Composition 1 | T0 | clear pale yellow soln | 7.47 | 98.82 | 97.50 | 0.15 |
| 2% PVP/1%PS80/1%PEG400 | T3 | clear pale yellow soln | 7.34 | 95.61 | 93.84 | 3.61 |
| Composition 2 | T0 | clear pale yellow soln | 7.44 | 98.94 | 96.03 | 0.15 |
| 2% PVP/1%PS80 /0.5%PEG400 | T3 | clear pale yellow soln | 7.29 | 94.69 | 93.18 | 4.62 |
| Composition 3 | T0 | soln w ppt | 7.51 | 98.73 | 99.83 | 0.16 |
| 2% PVP/1%MYS40/1% | T0 (filtrate) | | | 98.90 | 84.55 | 0.13 |

(continued)

| Samples | Conditions | Appearance | pH | Purity % | %LS | Compound II %area |
|---|---|---|---|---|---|---|
| PEG400 | T3 | soln w ppt | 7.25 | 94.91 | 92.28 | 4.38 |
| Composition 4 | T0 | clear pale yellow soln | 7.36 | 98.75 | 97.62 | 0.22 |
| 2% PVP/1%Tylox/1%PEG400 | T3 | soln w ppt | 7.29 | 94.92 | 91.35 | 4.30 |
| Composition 5 | T0 | soln w few particles | 7.42 | 98.71 | 96.88 | 0.18 |
| 2% PVP/0.5%PS80/ | T0 (filtrate) | | 7.42 | 98.98 | 96.05 | 0.15 |
| 1%PEG400 | T3 | soln w ppt | 7.32 | 94.66 | 92.97 | 4.47 |
| Composition 6 | T0 | clear pale yellow soln | 7.51 | 98.74 | 98.21 | 0.19 |
| 5%PVP/ 0.5%PS80/1%PEG400 | T3 | clear yellow soln | 7.19 | 94.81 | 95.44 | 4.22 |
| Composition 7 | T0 | clear pale yellow soln | 7.42 | 98.67 | 94.99 | 0.18 |
| 5% PVP/1%PS80/1%PEG400 | T3 | clear yellow soln | 7.14 | 94.34 | 95.24 | 4.64 |
| Composition 8 | T0 | clear pale yellow soln | 7.48 | 99.01 | 98.75 | 0.16 |
| 5% PVP/ 1%MYS40/1%PEG400 | T3 | clear yellow soln | 7.17 | 94.42 | 94.96 | 4.54 |
| Composition 9 | T0 | clear pale yellow soln | 7.44 | 98.79 | 96.49 | 0.16 |
| 5% PVP/1%Tylox/1%PEG400 | T3 | clear yellow soln | 7.21 | 95.29 | 95.08 | 3.81 |
| Composition 10 | T0 | clear pale yellow soln | 7.48 | 98.71 | 94.79 | 0.22 |
| 10% PVP/ 0.5%PS80/1%PEG400 | T3 | clear yellow soln | 7.06 | 95.10 | 94.46 | 3.83 |
| Composition 11 | T0 | clear pale yellow soln | 7.42 | 98.69 | 95.90 | 0.19 |
| 10% PVP/ 1%PS80/1%PEG400 | T3 | clear yellow soln | 7.05 | 94.97 | 94.52 | 3.93 |
| Composition 12 | T0 | clear pale yellow soln | 7.41 | 98.82 | 95.96 | 0.2 |
| 10% PVP/ 1%MYS40/1%PEG400 | T3 | clear yellow soln | 7.01 | 94.93 | 94.29 | 4.02 |
| Composition 13 | T0 | clear pale yellow soln | 7.44 | 98.83 | 95.52 | 0.19 |
| 10%PVP/1%Tylox/1%PEG400 | T3 | clear yellow soln | 7.03 | 94.98 | 94.73 | 3.95 |

This one embodiment provided results supporting the following conclusions. First, it was determined that at least 5% PVP K30 is needed to maintain the stability of compound I at 10 mM (concentration calculated based on compound I, not compound I choline salt). Also, surfactants did prove to be useful in maintaining solubility of compound I choline salt and/or compound II; however, PS 80 and tyloxapol were more effective than MYS40. The pH of the composition embodiments was not maintained using a 10 mM buffer; therefore it was determined that the buffer concentration should be increased in the final composition. Additionally, this particular embodiment aided in providing an appropriate method for determining stressing conditions that would potentially produce an amount of compound II substantially equivalent to that which would be formed in a composition having been stored for two years at room temperature.

In another embodiment, the buffer concentration was increased and the amount of PVP K30 was maintained at 10%. A control sample comprising 5% PVP K30 was also used in order to confirm the results of the previous embodiment. In this other embodiment, the concentration of compound I ranged from about 2 mM to about 10 mM (concentration calculated based on compound I, not compound I choline salt), and the other components used in the composition included various combinations of PEG400, PS 80, and tyloxapol. The various embodiments of the composition are provided in Table 7.

**Table 7: Prepared compositions for evaluation**

| F# | Compound I mM | PVP K30, %w/v | PS80, %w/v | Tylox, %/w/v | PEG400, %w/v | PG, %w/v | Phosphate buffer 25 mM |
|---|---|---|---|---|---|---|---|
| 1 | 10 | 5 | 1 | | 1 | 1.35 | qs |
| 2 | 10 | 10 | 0.5 | | 1 | 1.37 | qs |
| 3 | 10 | 10 | 1 | | 1 | 1.34 | qs |
| 4 | 10 | 10 | | 1 | 1 | 1.38 | qs |
| 5 | 10 | 10 | 1 | | | | qs(tonicity with NaCl) |
| 6 | 10 | 10 | | | 2 | 1.20 | qs |
| 7 | 10 | 10 | | | | 1.58 | qs |
| 8 | 2 | 10 | 1 | | 1 | 1.46 | qs |

In this other embodiment, the various composition embodiment were subjected to various temperature cycling studies to determine the composition's stability and capacity to tolerate extreme conditions. For example, 10 cycles of freeze/thaw (-20° C to RT), 8 cycles of freezing/heating (-20°C to 50°C) and 10 cycles of cooling/heating (5°C to 40°C) were used. Two samples, Samples 3 and 4, were stressed at 80 °C for approximately seven days prior to being exposed to the various temperature cycling studies. Surprisingly, many of the compositions comprising 10 mM of compound I (concentration calculated based on compound I, not compound I choline salt) exhibited no precipitation and contained only about 7% (about 290 μg/mL) of compound II after the temperature cycling studies (Table 8). Additionally, Samples 3 and 4 remained as clear yellow solutions with no precipitation even after the prior stressing conditions (Table 9). The important results obtained in this particular embodiment suggest that a suitable composition for use as disclosed herein could contain PVP, PS 80, tyloxapol, and PEG400, with the amount of PVP being greater than 5%.

**Table 8: Analytical results of prepared compositions after temperature cycling studies**

| Sample | LS mg/ml | Storage Condition | Appearance | pH | Purity % | % LS | Compound II % area |
|---|---|---|---|---|---|---|---|
| | | | | | | | |
| **Formulation 1** | 4.83 | TO | Clear yellow soln | 7.53 | 98.86 | 99.1 | 0.14 |
| 5% PVP K30/1%PS80/1%PEG400/1.5%PG | | (-)20-RT (5c) | Clear yellow soln | 7.46 | 98.99 | 99.7 | 0.17 |
| 25 mM Phosphate buffer | | (-)20-RT-(10c) | PPT | | | | |
| | | | | | | | |
| | | 5C-40C (5c) | Clear yellow soln | 7.41 | 98.64 | 99.6 | 0.30 |
| | | 5C-40C (10c) | PPT | | | | |
| | | | | | | | |
| | | (-)20-50C (5c) | Clear yellow soln | 7.45 | 98.38 | 99.1 | 0.54 |
| | | (-)20-50C (8c) | PPT | | | | |
| | | | | | | | |
| **Formulation 2** | 4.85 | TO | Clear yellow soln | 7.55 | 98.98 | 99.4 | 0.17 |
| 10% PVP K30/0.5%PS80/1%PEG400/1.4%PG | | (-)20-RT (5c) | Clear yellow soln | 7.47 | 98.81 | 98.8 | 0.20 |
| 25 mM Phosphate buffer | | (-)20-RT (10c) | Clear yellow soln | 7.47 | 98.92 | 97.4 | 0.21 |
| | | | | | | | |
| | | 5C-40C (5c) | Clear yellow soln | 7.42 | 98.66 | 99.0 | 0.38 |
| | | 5C-40C (10c) | Clear yellow soln | 7.40 | 98.50 | 96.1 | 0.46 |
| | | | | | | | |
| | | (-)20-50C (5c) | Clear yellow soln | 7.38 | 98.19 | 98.7 | 0.65 |
| | | (-)20-50C (8c) | Clear yellow soln | 7.37 | 97.96 | 95.7 | 0.95 |
| | | | | | | | |
| **Formulation 3** | 4.82 | TO | Clear yellow soln | 7.52 | 98.95 | 99.5 | 0.15 |
| 10% PVP K30/1%PS80/1%PEG400/1.3%PG | | (-)20-RT (5c) | Clear yellow soln | 7.46 | 99.02 | 99.2 | 0.18 |
| 25 mM Phosphate buffer | | (-)20-RT (10c) | Clear yellow soln | 7.42 | 98.88 | 96.9 | 0.24 |
| | | | | | | | |

(continued)

| | | | | | | | |
|---|---|---|---|---|---|---|---|
| **Formulation 3** | 4.82 | TO | Clear yellow soln | 7.52 | 98.95 | 99.5 | 0.15 |
| | | 5C-40C (5c) | Clear yellow soln | 7.42 | 98.66 | 99.3 | 0.37 |
| | | 5C-40C (10c) | Clear yellow soln | 7.38 | 98.56 | 95.9 | 0.49 |
| | | | | | | | |
| | | (-)20-50C (5c) | Clear yellow soln | 7.38 | 98.35 | 99.5 | 0.63 |
| | | | | | | | |
| **Formulation 4** | 4.82 | TO | Clear yellow soln | 7.60 | 98.92 | 99.9 | 0.15 |
| 10% PVP K30/1%Tyloxapol/1%PEG400/1.4% PG | | (-)20-RT (5c) | Clear yellow soln | 7.51 | 98.81 | 99.1 | 0.19 |
| 25 mM Phosphate buffer | | (-)20-RT (10c) | Clear yellow soln | 7.56 | 98.90 | 96.6 | 0.22 |
| | | 5C-40C (5c) | Clear yellow soln | 7.47 | 98.64 | 99.8 | 0.39 |
| | | 5C-40C (10c) | Clear yellow soln | 7.48 | 98.51 | 96.0 | 0.50 |
| | | | | | | | |
| | | (-)20-50C (5c) | Clear yellow soln | 7.42 | 98.13 | 101.2 | 0.66 |
| | | (-)20-50C (8c) | Clear yellow soln | 7.40 | 98.04 | 95.8 | 0.92 |
| | | | | | | | |
| **Formulation 5** | 4.81 | TO | Clear yellow soln | 7.49 | 98.97 | 98.6 | 0.14 |
| 10% PVP K30/1%PS80/0.6%NaCl | | (-)20-RT (5c) | Clear yellow soln | 7.44 | 98.92 | 98.1 | 0.18 |
| 25 mM Phosphate buffer | | (-)20-RT (10c) | Clear yellow soln | 7.46 | 98.81 | 97.4 | 0.25 |
| | | | | | | | |
| | | 5C-40C (5c) | Clear yellow soln | 7.36 | 98.62 | 98.9 | 0.36 |
| | | 5C-40C (10c) | Clear yellow soln | 7.38 | 98.54 | 96.0 | 0.48 |
| | | | | | | | |
| | | (-)20-50C (5c) | Clear yellow soln | 7.31 | 98.16 | 98.4 | 0.67 |
| | | (-)20-50C (8c) | Clear yellow soln | 7.32 | 97.90 | 94.6 | 0.98 |
| | | | | | | | |

(continued)

| | | | | | | | |
|---|---|---|---|---|---|---|---|
| **Formulation 6** | 4.78 | TO | Clear yellow soln | 7.52 | 98.96 | 98.8 | 0.15 |
| 10% PVP K30/2%PEG400/1.2%PG | | (-)20-RT (5c) | Clear yellow soln | 7.45 | 98.96 | 98.8 | 0.19 |
| 25 mM Phosphate buffer | | (-)20-RT (10c) | Clear yellow soln | 7.43 | 98.88 | 97.1 | 0.23 |
| | | | | | | | |
| | | 5C-40C (5c) | Clear yellow soln | 7.41 | 98.70 | 98.9 | 0.37 |
| | | 5C-40C (10c) | Clear yellow soln | 7.38 | 98.59 | 96.0 | 0.49 |
| | | | | | | | |
| | | (-)20-50C (5c) | Clear yellow soln | 7.35 | 98.15 | 99.2 | 0.68 |
| | | | | | | | |
| **Formulation** 7 | 4.82 | TO | Clear yellow soln | 7.48 | 98.96 | 99.2 | 0.16 |
| 10% PVP K30/1.6%PG | | (-)20-RT (5c) | Clear yellow soln | 7.43 | 98.86 | 99.2 | 0.15 |
| 25 mM Phosphate buffer | | (-)20-RT (10c) | Clear yellow soln | 7.40 | 99.01 | 98.4 | 0.20 |
| | | | | | | | |
| | | 5C-40C (5c) | Clear yellow soln | 7.39 | 98.64 | 98.9 | 0.33 |
| | | 5C-40C (10c) | Clear yellow soln | 7.35 | 98.66 | 96.1 | 0.47 |
| | | | | | | | |
| | | (-)20-50C (5c) | Clear yellow soln | 7.36 | 98.32 | 98.8 | 0.59 |
| | | (-)20-50C (8c) | Clear yellow soln | 7.30 | 98.01 | 94.8 | 0.95 |
| | | | | | | | |
| **Formulation 8** | 0.96 | TO | Clear yellow soln | 7.51 | 98.79 | 99.1 | 0.18 |
| 10% PVP K30/1%PS80/1%PEG400/1.6%PG | | (-)20-RT (5c) | Clear yellow soln | 7.45 | 98.60 | 99.5 | 0.18 |
| 25 mM Phosphate buffer | | (-)20-RT (10c) | Clear yellow soln | 7.41 | 98.55 | 96.1 | 0.25 |
| | | | | | | | |
| | | 5C-40C (5c) | Clear yellow soln | 7.38 | 98.40 | 98.2 | 0.35 |
| | | 5C-40C (10c) | Clear yellow soln | 7.35 | 98.23 | 95.0 | 0.52 |
| | | | | | | | |

(continued)

| | | | | | | | |
|---|---|---|---|---|---|---|---|
| **Formulation 8** | 0.96 | TO | Clear yellow soln | 7.51 | 98.79 | 99.1 | 0.18 |
| | | (-)20-50C (5c) | Clear yellow soln | 7.32 | 97.87 | 97.4 | 0.64 |
| | | (-)20-50C (8c) | Clear yellow soln | 7.35 | 97.81 | 93.4 | 0.96 |

**Table 9: Analytical results of compositions #3 and #4 after stressing at 80 °C for 7 days and 10 cycles of temperature cycling**

| Sample | Storage condition | Appearance | pH | Purity % | %LS | Compound II %area | Compound II µg/ml |
|---|---|---|---|---|---|---|---|
| | | | | | | | |
| **Formulation 3** | 5C-40C | Clear yellow soln | 7.17 | 91.5 | 87.4 | 7.27 | 300.0 |
| 10% PVP K30/1%PS80/1%PEG400/1.3%PG | (-)20-RT | Clear yellow soln | 7.14 | 91.7 | 87.3 | 7.15 | 294.1 |
| 25 mM Phosphate buffer | (-)20-50C | Clear yellow soln | 7.13 | 91.4 | 87.3 | 7.41 | 305.1 |
| | | | | | | | |
| **Formulation 4** | 5C-40C | Clear yellow soln | 7.17 | 92.1 | 87.5 | 6.92 | 283.7 |
| 10% PVP K30/1%Tyloxapol/1%PEG400/1.4% PG | (-)20-RT | Clear yellow soln | 7.16 | 92.2 | 87.5 | 6.89 | 282.5 |
| 25 mM Phosphate buffer | (-)20-50C | Clear yellow soln | 7.19 | 91.7 | 87.2 | 7.2 | 295.4 |

In yet another embodiment, the effects of adding other components to the disclosed composition were determined. In particular embodiments of the disclosed composition, a nucleation inhibitor was added. Nucleation inhibitors are added to evaluate whether they decreased the amount of precipitation of compound I choline salt and/or compound II in certain composition embodiments. The sample compositions analyzed in this embodiment are provided in Table 10.

**Table 10: Prepared compositions for evaluation**

| F# | Compound ImM | PVP K30, %w/v | HPMC E5, w/v% | PS80, %w/v | Tylox, %/w/v | PEG40 0, %w/v | PG, %w/v | Phosphate buffer 25 mM |
|---|---|---|---|---|---|---|---|---|
| 1 | 2 | 7.5 | | 1 | | 1 | 1.46 | qs |
| 2 | 10 | 7.5 | | 1 | | 1 | 1.34 | qs |
| 3 | 10 | 7.5 | | 0.5 | | 1 | 1.37 | qs |
| 4 | 10 | 7.5 | | 1 | | | | qs(tonicity with NaCl) |
| 5 | 10 | 7.5 | | | 1 | 1 | 1.38 | qs |
| 6 | 10 | 7.5 | | | 1 | | 1.57 | qs |
| 7 | 10 | 7.5 | | | 0.5 | | 1.58 | qs |
| 8 | 10 | 7.5 | | | | | 1.59 | qs |
| 9 | 10 | 10 | | 0.5 | | | 1.56 | qs |
| 10 | 10 | 10 | | | 0.5 | | 1.70 | qs |
| 11 | 2 | 10 | | | | | 1.58 | qs |
| 12 | 10 | | 1 | 1 | | | 1.54 | qs |
| 13 | 10 | | 1 | | 1 | | 1.58 | qs |
| 14 | 10 | | 1 | 0.5 | | | 1.57 | qs |
| 15 | 10 | | 1 | | 0.5 | | 1.59 | qs |
| 16 | 10 | | 1 | | | | 1.60 | qs |
| 17 | 10 | 10 | | | 1 | | 1.57 | qs |

Surprisingly, sample compositions comprising 1% of the nucleation inhibitor, HPMC E5, exhibited precipitation after merely storing at room temperature overnight. Further analysis of these samples (Samples 12-16) was not carried out. Samples 1-11 and 17 were further evaluated by stressing at 60 °C for 14 days and 80 °C for six days. Subsequently, these samples were subjected to 5 cycles of freezing/heating (-20 °C to 50 °C). These samples exhibited no precipitation and remained as clear yellow solutions.

From this further embodiment, it was determined that a particular amount of an HPMC nucleation inhibitor surprisingly did not aid in maintaining the physical and/or chemical properties of the disclosed composition. Additionally, these embodiments of the disclosed composition indicated that the amount of PVP could be reduced to about 7.5%, even when PEG400 was not present. Accordingly, this other embodiment provided a basis for determining a potential minimum PVP amount suitable for use in the disclosed composition in combination with the fewest number of excipients.

In yet another embodiment, the ability of a nucleation inhibitor, HPMC E5, to aid in maintaining the chemical and/or physical properties of the composition were again analyzed; however, the amount of nucleation inhibitor added to the composition was decreased to about 0.25%. The other components used in this particular embodiment are provided in Table 11.

**Table 11: Prepared compositions for evaluation**

| F# | Compound I mM | PVP K30, %w/v | HPMC E5 w/v% | PS80, %w/v | Tylox, %/w/v | PG, %w/v | Phosphate buffer, 25 mM |
|---|---|---|---|---|---|---|---|
| 1 | 10 | 7.5 | 0.25 | | | 1.59 | qs |
| 2 | 10 | 10 | 0.25 | | | 1.58 | qs |
| 3 | 10 | 7.5 | 0.25 | 0.5 | | 1.56 | qs |
| 4 | 10 | 10 | 0.25 | 0.5 | | 1.55 | qs |
| 5 | 10 | 10 | 0.25 | 1 | | 1.52 | qs |
| 6 | 12.5 | 10 | | 1 | | 1.49 | qs |
| 7 | 12.5 | 10 | 0.25 | 1 | | 1.49 | qs |

(continued)

| F# | Compound I mM | PVP K30, %w/v | HPMC E5 w/v% | PS80, %w/v | Tylox, %/w/v | PG, %w/v | Phosphate buffer, 25 mM |
|---|---|---|---|---|---|---|---|
| 8 | 12.5 | 10 | | | 1 | 1.53 | qs |
| 9 | 12.5 | 10 | 0.25 | | 1 | 1.53 | qs |

The various sample compositions examined in this particular embodiment were subjected to stressing at 60 °C for 14 days and then stored at room temperature for at least four weeks. Again, the samples comprising 0.25% of the nucleation inhibitor exhibited precipitation after stressing at 60 °C for 14 days. Thus, nucleation inhibitors did not appear to be a viable option for maintaining the chemical and/or physical properties of the disclosed composition, even in small amounts. This further embodiment also provided the surprising result that higher concentrations of compound I could be used without a necessary corresponding increase in the amount of PVP K30 provided and that other excipients were unnecessary in order to maintain solubility of compound I choline salt and/or compound II in certain composition embodiments.

Another round of compositions was tested wherein the amount of compound I was maintained at 10 mg/mL (concentration calculated based on compound I, not compound I choline salt). Stressing at 80 °C was carried out for 6 days, followed by 5 cycles of cooling/heating (about 5 °C to about 40 °C). The disclosed compositions all remained as clear yellow solutions. The particular components of this embodiment are provided in Table 12.

**Table 12: Prepared compositions for evaluation**

| F# | Compound I mg/mL | PVP K30, %w/v | PS80, %w/v | Tylox, %/w/v | PG, %w/v | Phosphate buffer, 25 mM |
|---|---|---|---|---|---|---|
| 1 | 10 | 7.5 | | | 1.45 | qs |
| 2 | 10 | 10 | | | 1.42 | qs |
| 3 | 10 | 10 | 1 | | 1.36 | qs |
| 4 | 10 | 10 | | 0.5 | 1.41 | qs |

Based on the findings of these various composition testing rounds, exemplary embodiments of the disclosed formation were determined and are provided in Table 13, below. Particular composition embodiments can comprise from about 1 mg/mL to about 10 mg/mL of compound I, or its choline salt. Exemplary embodiments concern using 2 mg/mL or 5 mg/mL of a compound I choline salt.

**Table 13: Compound I ophthalmic prototype compositions**

| F# | Compound I eq, mg/mL | PVP K30 %w/v | PS80 %w/v | Tylox %/w/v | PG %w/v | Phosphate buffer 25 mM |
|---|---|---|---|---|---|---|
| 1 | 1, 5 | 10 | 1 | | v | qs |
| 2 | 1, 5, 10 | 10 | | | v | qs |
| 3 | 1,5,10 | 7.5 | | | v | qs |
| 4 | 1, 5 | 10 | | 0.5 | v | qs |
| 5 | 10 | 10 | | 0.3 | v | qs |

**Table 14: Additional composition embodiments**

| Component | Grade | Weight% (w/v) | Weight (g) per 1000 mL[b] |
|---|---|---|---|
| Povidone K30 | USP, Ph. Eur. | 7.5 | 75 |
| Monobasic Sodium Phosphate, Monohydrate | USP, Ph. Eur. | 0.065 | 0.65 |
| Dibasic Sodium Phosphate, Anhydrate | USP, Ph. Eur. | 0.287 | 2.87 |
| Propylene Glycol [a] | USP | 1.6 | 16 |
| Purified Water | USP | QS | QS |
| | **TOTAL** | **100.0** | **1025** |

[a] Propylene Glycol is used as a tonicity agent to adjust osmolality of formulation to 275 +/- 45 mOsm/kg.
[b]The density of the placebo is 1.0246 g/mL at 20-25°C.

**Table 15: Additional composition embodiments**

| Component | Grade | Weight% (w/v) | Weight (g) Per 1000 mL[c] |
|---|---|---|---|
| Compound I[a] | GMP | 1.0 | 10 |
| Povidone K30 | USP, Ph. Eur. | 7.5 | 75 |
| Monobasic Sodium Phosphate, Monohydrate | USP, Ph. Eur. | 0.065 | 0.65 |
| Dibasic Sodium Phosphate, Anhydrate | USP, Ph. Eur. | 0.287 | 2.87 |
| Propylene Glycol [b] | USP | 1.4 | 14 |
| Purified Water | USP | QS | QS |
| | **TOTAL** | **100.0** | **1028** |

[a] The API is Compound I Choline. The quantity of API should be adjusted according to the weight purity of a given lot. 1.213 mg of Compound I choline = 1 mg of Compound I.
[b] Propylene Glycol is used as a tonicity agent to adjust osmolality of formulation to 275 +/- 45 mOsm/kg
[c] The density of the formulation is 1.0284 g/mL at 20-25°C.

**Table 16: Additional composition embodiments**

| Component | Grade | Weight% (w/v) | Weight (g) Per 1000 mL[c] |
|---|---|---|---|
| Compound I[a] | GMP | 0.5 | 5 |
| Povidone K30 | USP, Ph. Eur. | 7.5 | 75 |
| Monobasic Sodium Phosphate, Monohydrate | USP, Ph. Eur. | 0.065 | 0.65 |
| Dibasic Sodium Phosphate, Anhydrate | USP, Ph. Eur. | 0.287 | 2.87 |
| Propylene Glycol [b] | USP | 1.5% | 15 |
| Purified Water | USP | QS | QS |
| | **TOTAL** | **100.0** | **1027** |

[a] The API is Compound I Choline. The quantity of API should be adjusted according to the weight purity of a given lot. 1.213 mg of Compound I choline = 1 mg of Compound I.
[b] Propylene Glycol is used as a tonicity agent to adjust osmolality of formulation to 275 +/- 45 mOsm/kg
[c] The density of the formulation is 1.0269 g/mL at 20-25°C.

**Table 17: Additional composition embodiments**

| Component | Grade | Weight% (w/v) | Weight (g) Per 1000 mL[c] |
|---|---|---|---|
| Compound I[a] | GMP | 0.2 | 2 |
| Povidone K30 | USP, Ph. Eur. | 7.5 | 75 |
| Monobasic Sodium Phosphate, Monohydrate | USP, Ph. Eur. | 0.065 | 0.65 |
| Dibasic Sodium Phosphate, Anhydrate | USP, Ph. Eur. | 0.287 | 2.87 |
| Propylene Glycol [b] | USP | 1.54 | 15.4 |
| Purified Water | USP | QS | QS |
| | **TOTAL** | **100.0** | **1027** |

[a] The API is Compound I Choline. The quantity of API must be adjusted according to the weight purity of a given lot. 1.213 mg of Compound I choline = 1 mg of Compound I.
[b] Propylene Glycol is used as a tonicity agent to adjust osmolality of formulation to 275 +/- 45 mOsm/kg
[c] The density of the formulation is 1.0268 g/mL at 20-25°C.

**Assay for Ramos B-Cell Line Stimulated with IL-4**

One means of assaying for JAK inhibition is detection of the effect of compounds I and II on the upregulation of downstream gene products. In the Ramos/IL4 assay, B-cells are stimulated with the cytokine Interleukin-4 (IL-4)

leading to the activation of the JAK/Stat pathway through phosphorylation of the JAK family kinases, JAK1 and JAK3, which in turn phosphorylate and activate the transcription factor Stat-6. One of the genes upregulated by activated Stat-6 is the low affinity IgE receptor, CD23. To study the effect of inhibitors (for example, the 2,4-substituted pyrimidinediamine compounds described herein) on the JAK1 and JAK3 kinases, human Ramos B cells are stimulated with human IL-4. Twenty to 24 hours post stimulation, cells are stained for upregulation of CD23 and analyzed using flow cytometry (FACS). A reduction of the amount of CD23 present compared to control conditions indicates the test compound actively inhibits the JAK kinase pathway. An exemplary assay of this type is described in greater detail below.

B-cells stimulated with cytokine Interleukin-4 (IL-4) activate the JAK/Stat pathway through phosphorylation of the JAK family kinases, JAK-1 and JAK-3, which in turn phosphorylate and activate the transcription factor Stat-6. One of the genes upregulated by activated Stat-6 is the low affinity IgE receptor, CD23. To study the effect of inhibitors on the JAK family kinases, human Ramos B cells are stimulated with human IL-4.

The Ramos B-cell line was acquired from ATCC (ATCC Catalog No. CRL-1596). The cells were cultured in RPMI 1640 (Cellgro, MediaTech, Inc., Herndon, VA, Cat No. 10-040-CM) with 10 % fetal bovine serum (FBS), heat inactivated (JRH Biosciences, Inc., Lenexa, Kansas, Cat No. 12106-500M) according to ATCC propagation protocol. Cells were maintained at a density of 3.5 x $10^5$. The day before the experiment, Ramos B-cells were diluted to 3.5 x $10^5$ cells/mL to ensure that they were in a logarithmic growth phase.

Cells were spun down and suspended in RPMI with 5% serum. 5 x $10^4$ cells were used per point in a 96-well tissue culture plate. Cells were pre-incubated with compound or DMSO (Sigma-Aldrich, St. Louis, MO, Cat No. D2650) vehicle control for 1 hour in a 37 °C incubator. Cells were then stimulated with IL-4 (Peprotech Inc., Rocky Hill, NJ, Cat No. 200-04) for a final concentration of 50 units/mL for 20-24 hours. Cells were then spun down and stained with anti-CD23-PE(BD Pharmingen, San Diego, CA, Cat No. 555711) and analyzed by FACS. Detection was performed using a BD LSR I System Flow Cytometer, purchased from Becton Dickinson Biosciences of San Jose, California. The $IC_{50}$ calculated based on the results of this assay are provided in Table 18.

**Primary Human T-cell Proliferation Assay Stimulated with IL-2**

The JAK activity of compounds I and/or II (or a pharmaceutically acceptable salt, solvate, or *N*-oxide thereof), described herein, may further be characterized by assaying the effect of these compounds described herein on the proliferative response of primary human T-cells. In this assay, primary human T-cells derived from peripheral blood and pre-activated through stimulation of the T-cell receptor and CD28, proliferate in culture in response to the cytokine Interleukin-2 (IL-2). This proliferative response is dependent on the activation of JAK1 and JAK3 tyrosine kinases, which phosphorylate and activate the transcription factor Stat-5. The primary human T-cells are incubated with compounds I and/or II (or a pharmaceutically acceptable salt, solvate, or *N*-oxide thereof) in the presence of IL-2 for 72 hours and at the assay endpoint intracellular ATP concentrations are measured to assess cell viability. A reduction in cell proliferation compared to control conditions is indicative of inhibition of the JAK kinase pathway. An exemplary assay of this type is described in greater detail below.

Primary human T-cells derived from peripheral blood and pre-activated through stimulation of the T-cell receptor and CD28, proliferate *in vitro* in response to the cytokine Interleukin-2 (IL-2). This proliferative response is dependent on the activation of JAK-1 and JAK-3 tyrosine kinases, which phosphorylate and activate the transcription factor Stat-5.

Human primary T cells were prepared as follows. Whole blood was obtained from a healthy volunteer, mixed 1:1 with PBS, layered on to Ficoll Hypaque (Amersham Pharmacia Biotech, Piscataway, NJ, Catalog #17-1440-03) in 2:1 blood/PBS:ficoll ratio and centrifuged for 30 minutes at 4 °C at 1750 rpm. The lymphocytes at the serum: ficoll interface were recovered and washed twice with 5 volumes of PBS. The cells were resuspended in Yssel's medium (Gemini Bio-products, Woodland, CA, Catalog #400-103) containing 40 U/mL recombinant IL2 (R and D Systems, Minneapolis, MN, Catalog #202-IL (20 $\mu$g)) and seeded into a flask pre-coated with 1 $\mu$g/mL anti-CD3 (BD Pharmingen, San Diego, CA, Catalog #555336) and 5 $\mu$g/mL anti-CD28 (Immunotech, Beckman Coulter of Brea California, Catalog #IM1376). The primary T- cells were stimulated for 3-4 days, then transferred to a fresh flask and maintained in RPMI with 10% FBS and 40 U/mL IL-2.

Primary T-cells were washed twice with PBS to remove the IL-2 and resuspended in Yssel's medium at 2 x $10^6$ cells/mL. 50 $\mu$L of cell suspension containing 80 U/mL IL-2 was added to each well of a flat bottom 96 well black plate. For the unstimulated control, IL-2 was omitted from the last column on the plate. Compounds were serially diluted in dimethyl sulfoxide (DMSO, 99.7% pure, cell culture tested, Sigma-Aldrich, St. Louis, MO, Catalog No. D2650) from 5 mM in 3-fold dilutions, and then diluted 1:250 in Yssel's medium. 50 $\mu$E of 2X compound was added per well in duplicate and the cells were allowed to proliferate for 72 hours at 37 °C.

Proliferation was measured using CellTiter-Glo® Luminescent Cell Viability Assay (Promega), which determines the number of viable cells in culture based on quantitation of the ATP present, as an indicator of metabolically active cells. The substrate was thawed and allowed to come to room temperature. After mixing the Cell Titer-Glo reagent and diluent together, 100 $\mu$L was added to each well. The plates were mixed on an orbital shaker for two minutes to induce

lysis and incubated at room temperature for an additional ten minutes to allow the signal to equilibrate. Detection was performed using a Wallac Victor2 1420 multilabel counter purchased from Perkin Elmer, Shelton, CT. The $IC_{50}$ calculated based on the results of this assay are provided in Table 18.

**A549 Epithelial Line Stimulated with IFNγ**

The JAK activity of compounds I and/or II (or a pharmaceutically acceptable salt, solvate, or *N*-oxide thereof) described herein may also be characterized by assaying the effect of these compounds on A549 lung epithelial cells and U937 cells. A549 lung epithelial cells and U937 cells up-regulate ICAM-1 (CD54) surface expression in response to a variety of different stimuli. Therefore, using ICAM-1 expression as readout, test compound effects on different signaling pathways can be assessed in the same cell type. Stimulation with IL-1β through the IL-1β receptor activates the TRAF6 / NPκB pathway resulting in up-regulation of ICAM-1. IFNγ induces ICAM-1 up-regulation through activation of the JAK1/JAK2 pathway. The up-regulation of ICAM-1 can be quantified by flow cytometry across a compound dose curve and $EC_{50}$ values are calculated. Exemplary assays of this type are described in greater detail below.

A549 lung epithelial cells up-regulate ICAM-1 (CD54) surface expression in response to a variety of different stimuli. Therefore, using ICAM-1 expression as readout, compound effects on different signaling pathways can be assessed in the same cell type. IFNγ up-regulates ICAM-1 through activation of the JAK/Stat pathway. In this example, the up-regulation of ICAM-1 by IFNγ was assessed.

The A549 lung epithelial carcinoma cell line originated from the American Type Culture Collection. Routine culturing was with F12K media (Mediatech Inc., Lenexa, KS, Cat. No. 10-025-CV) with 10% fetal bovine serum, 100 I.U. penicillin and 100 ng/mL streptomycin (complete F12k media). Cells were incubated in a humidified atmosphere of 5% $CO_2$ at 37 °C. Prior to use in the assay, A549 cells were washed with PBS and trypsinized (Mediatech Inc., Cat. No. 25-052-CI) to lift the cells. The trypsin cell suspension was neutralized with complete F12K media and centrifuged to pellet the cells. The cell pellet was resuspended in complete F12K media at a concentration of $2.0x10^5$/mL. Cells were seeded at 20,000 per well, 100 μL total volume, in a flat bottom tissue culture plate and allowed to adhere overnight.

On day two, A549 cells were pre-incubated with a 2,4-substituted pyrimidinediamine test compound or DMSO (control) (Sigma-Aldrich, St. Louis, MO, Catalog No. D2650) for 1 hour. The cells were then stimulated with IFNγ (75 ng/mL) (Peprotech Inc., Rocky Hill, NJ, Cat. No. 300-02) and allowed to incubate for 24 hours. The final test compound dose range was 30 μM to 14 nM in 200 μL F12K media containing 5% FBS, 0.3% DMSO.

On day three, the cell media was removed and the cells were washed with 200 μL PBS (phosphate buffered saline). Each well was trypsinized to dissociate the cells, then neutralized by addition of 200 μL complete F12K media. Cells were pelleted and stained with an APC conjugated mouse anti-human ICAM-1 (CD54) (BD Pharmingen, San Diego, CA, Catalog #559771) antibody for 20 minutes at 4 °C. Cells were washed with ice cold FACS buffer (PBS + 2% FBS) and surface ICAM-1 expression was analyzed by flow cytometry. Detection was performed using a BD LSR I System Flow Cytometer, purchased from BD Biosciences of San Jose, California. Events were gated for live scatter and the geometric mean was calculated (Becton-Dickinson CellQuest software version 3.3, Franklin Lakes, NJ). Geometric means were plotted against compound concentration to generate a dose response curve. The $IC_{50}$ calculated based on the results of this assay are provided in Table 18.

**U937 IFNγ ICAM1 FACS Assay**

U937 human monocytic cells up-regulate ICAM-1 (CD54) surface expression in response to a variety of different stimuli. Therefore, using ICAM-1 expression as readout, compound effects on different signaling pathways can be assessed in the same cell type. IFNγ up-regulates ICAM-1 through activation of the JAK/Stat pathway. In this example, the up-regulation of ICAM-1 by IFNγ was assessed.

The U937 human monocytic cell line was obtained from ATCC of Rockville Maryland, catalog number CRL-1593.2, and cultured in RPM1-1640 medium containing 10% (v/v) FCS. U937 cells were grown in 10% RPMI. The cells were then plated at a concentration of 100,000 cells per 160 μL in 96 well flat bottom plates. The test compounds were then diluted as follows: 10 mM test compound was diluted 1:5 in DMSO (3μL 10 mM test compound in 12 μL DMSO), followed by a 1:3 serial dilution of test compound in DMSO (6 μL test compound serially diluted into 12 μL DMSO to give 3-fold dilutions). Then 4 μL of test compound was transferred to 76 μL of 10% RPMI resulting in a 10X solution (100 μM test compound, 5% DMSO). For control wells, 4 μL of DMSO was diluted into 76 μL 10% RPMI.

The assay was performed in duplicate with 8 points (8 3-fold dilution concentrations from 10 μl) and with 4 wells of DMSO only (control wells) under stimulated conditions and 4 wells of DMSO only under unstimulated conditions.

The diluted compound plate was mixed 2X using a multimek (Beckman Coulter of Brea, California) and then 20 μL of the diluted compounds was transferred to the 96 well plate containing 160 μL of cells, which were then mixed again twice at low speeds. The cells and compounds were then pre-incubated for 30 minutes at 37 °C with 5% $CO_2$.

The 10X stimulation mix was made by preparing a 100 ng/mL solution of human IFNγ in 10% RPMI. The cells

and compound were then stimulated with 20 μL of IFNγ stimulation mix to give a final concentration of 10 ng/mL IFNγ, 10 μM test compound, and 0.5% DMSO. The cells were kept under conditions for stimulation for 18-24 hours at 37 °C with 5% $CO_2$.

The cells were transferred to a 96 well round bottom plate for staining and then kept on ice for the duration of the staining procedure. Cells were spun down at 1000 rpm for 5 minutes at 4 °C, following which the supernatant was removed. Following removal of the supernatant, 1 μL APC conjugated mouse anti-human ICAM-1 antibody was added per 100 μL FACS buffer. The cells were then incubated on ice in the dark for 30 minutes. Following incubation, 150 μL of FACS buffer was added and the cells were centrifuged at 1000 rpm for 5 minutes at 4 °C, following which the supernatant was removed. After removal of the supernatant, 200 μL of FACS buffer was added and the cells were resuspended. After suspension, the cells were centrifuged at 1000 rpm for 5 min at 4 °C. Supernatant was then removed prior to resuspension of the cells in 150 μL FACS buffer.

Detection was performed using a BD LSR I System Flow Cytometer, purchased from BD Biosciences of San Jose, California. The live cells were gated for live scatter and the geometric mean of ICAM-APC was measured (Becton-Dickinson CellQuest software version 3.3, Franklin Lakes, NJ). Both % live cells and ICAM-1 expression was analyzed. The assays for the test compounds were carried out in parallel with a control compound of known activity. The $EC_{50}$ for the control compound is typically 40-100 nM. The $IC_{50}$ calculated based on the results of this assay are provided in Table 18.

**Table 18: Assay Results**

| Compound | Example 3 | Example 4 | Example 5 | Example 6 |
|---|---|---|---|---|
| I | 9.655 | | | |
| II | 0.056 | 0.181 | 11.338 | 0.565 |
| III | 3.972 | | | |
| IV | 2.318 | 5.560 | | |
| V | 0.373 | | | 25.126 |
| VI | 0.104 | 0.262 | 4.973 | 0.424 |
| VII | 0.022 | 0.053 | | 0.140 |

**Induced Dry Eye Mouse Model**

This example describes the treatment of symptoms in a mouse model of dry eye. An injection was prepared comprising 2.5 mg/mL scopolamine (Sigma-Aldrich) in injectable saline (1-1.5 mL per animal). Normal C57 mice are injected with 200-250 μL of scopolamine solution four times every 2.5 hours in alternating hindquarters. The mice are placed in special cages (with holes in front and back) and placed in a hood. Fans are placed in front of each cage and run for 16 hours overnight for five consecutive days. Measurements of tear production are taken daily and at the end of five days all mice are considered dry-induced. Animals may be treated with any of the composition embodiments disclosed herein at 1 μL, once per day for two weeks. Tear production is measured with treatment results being measured by restoration in part or in whole of normal tear production values.

Tear production can be measured either by quantitative means, or qualitative assessment of the animal's corneas can be performed, for example by biomicroscopic examination of fluorescein or rose bengal staining patterns of the treated eyes. A reduction in such staining is indicative of successful treatment of the dry eye condition.

**Other Animal Models**

Several models of Sjogren's syndrome have been developed that mimic the early activation and infiltration of autoreactive lymphocytes seen in that condition. The non-obese diabetic (NOD) mouse model shows a lymphocytic infiltration of predominantly $CD4^+$ Th1 cells in the lacrimal gland as well as other organs, including the pancreas, submandibular, and thyroid glands. Male NOD mice show significant inflammatory lesions of the lacrimal gland from the age of 8 weeks, whereas female NOD mice do not show any changes until 30 weeks of age. Takahashi et al., High incidence of autoimmune dacryoadenitis in male non-obese diabetic (NOD) mice depending on sex steroid. Clin Exp Immunol. 1997;109:555-561. The MRL/MpJ-$fas^+/fas^+$ (MRL/+) and MRL/MpJ-$fas^{lpr}/fas^{lpr}$ (MRL/1pr) mouse models of Sjögren's syndrome exhibit lacrimal gland infiltrates characterized by a predominance of $CD4^+$ T cells. Van Blokland and Versnel, Pathogenesis of Sjögren's syndrome: characteristics of different mouse models for autoimmune exocrinopathy. Clin Immunol. 2002;103:111-124.

Because of the large exposed ocular surface in rabbits compared with mice, standard dry eye clinical tests, such as tear break-up time and fluorescein or rose bengal staining of the ocular surface, are more easily performed in

rabbits. An autoimmune disease in rabbits resembling Sjögren's syndrome can be provoked by injecting into the lacrimal gland autologous peripheral blood lymphocytes proliferated in culture with epithelial cells obtained from the contralateral excised gland.

This and other animal models may be used to test the compositions disclosed herein. The disclosed compositions may be administered to the animal, and the eyes examined for evidence of increased tear production or decreased evidence of dry eyes.

In some embodiments, a subject is administered a composition comprising 7.5% w/v K30-grade polyvinylpyrrolidone, a phosphate buffer having a concentration of about 25 mM, and 1.6% w/v propylene glycol. The subject is selected because the subject is suffering one or more symptoms associated with dry eye syndrome, or because the subject has been diagnosed with dry eye syndrome. In particular disclosed embodiments, the dry eye syndrome from which the subject suffers (or exhibits symptoms) is treated, ameliorated, or prevented. For example, in some embodiments, the subject exhibits an improvement in the physical state of the subject's eyes (*e.g.*, increased tear production or decreased evaporation). In some embodiments, the subject demonstrates reduced inferior corneal fluorescein staining, improvement in a Schirmer test, or improvement in signs and/or symptoms of dry eye syndrome. Such improvement is measured using the Ocular Surface Disease Index, the Ocular Comfort Index, or similar tests.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims.

## Claims

1. A composition for ophthalmic administration, comprising:

   (i) 5% w/v to 15% w/v polyvinylpyrrolidone having an average molecular weight of from 40,000 to 60,000, and less than 400 ppm peroxide; and
   (ii) at least one compound selected from the group consisting of

   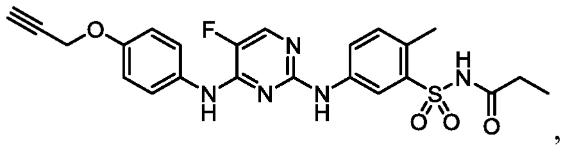
   ,

   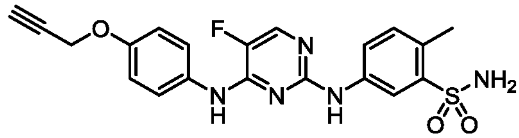
   ,

   and pharmaceutically acceptable salts, solvates, or *N*-oxides thereof.

2. The composition of claim 1, further comprising a buffer and a tonicity agent.

3. The composition of claim 2, wherein the buffer has a concentration ranging from about 20 mM to 30 mM.

4. The composition of claim 3, wherein the buffer is a phosphate buffer comprising monobasic sodium phosphate monohydrate and dibasic sodium phosphate anhydrate.

5. The composition of claim 1, having a pH ranging from 7.0 to 8.0.

6. The composition of claim 2, wherein the tonicity agent is present in an amount sufficient to provide osmolality of from 230 mOsm/kg to 320 mOsm/kg.

7. The composition of claim 2, wherein the tonicity agent is present in an amount ranging from 1% w/v to 2% w/v.

8. The composition of claim 2, wherein the tonicity agent is propylene glycol.

9. The composition of claim 1, further comprising at least one additional pharmacologically active compound.

10. The composition of claim 9, wherein the at least one additional pharmacologically active compound is capable of treating, ameliorating, and/or preventing an eye disease or disorder.

11. The composition of claim 9, wherein the at least one additional pharmacologically active compound is a corticosteroid, an antihistamine, an antibiotic, an anti-inflammatory, an antiviral, a glaucoma medication, or combinations thereof.

12. The composition of claim 1, further comprising a buffer having a concentration of 20 mM to 30 mM, and 1% w/v to 2% w/v of a tonicity agent.

13. The composition of claim 1, comprising 7.5% w/v polyvinylpyrrolidone K30, and further comprising a monobasic sodium phosphate monohydrate/dibasic sodium phosphate anhydrate buffer having a concentration of 25 mM, and 1.6% w/v propylene glycol.

14. The composition of claim 1, wherein the composition comprises a compound having the formula

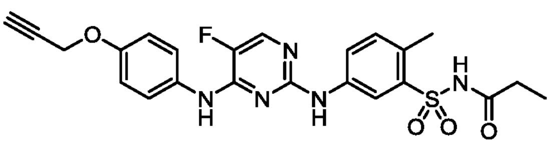

or a pharmaceutically acceptable salt, solvate or N-oxide thereof.

## Patentansprüche

1. Zusammensetzung für ophthalmische Verabreichung, umfassend:

(i) 5 % w/v bis 15 % w/v Polyvinylpyrrolidon mit einem Durchschnittsmolekulargewicht von 40.000 bis 60.000 und weniger als 400 ppm Peroxid; und
(ii) mindestens eine Verbindung aus der Gruppe bestehend aus

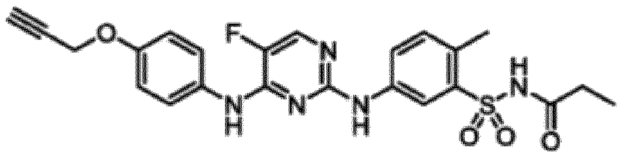,

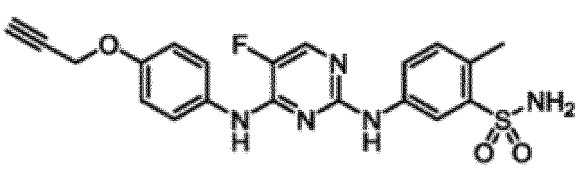,

und pharmazeutisch verträgliche Salze, Solvate oder *N*-Oxide davon.

2. Zusammensetzung nach Anspruch 1, ferner umfassend einen Puffer und ein Tonizitätsmittel.

3. Zusammensetzung nach Anspruch 2, wobei der Puffer eine Konzentration zwischen etwa 20 mM bis 30 mM hat.

4. Zusammensetzung nach Anspruch 3, wobei der Puffer ein Phosphatpuffer ist, der Natriumdihydrogenphosphat-Monohydrat und Dinatriumhydrogenphosphat-Anhydrat umfasst.

5. Zusammensetzung nach Anspruch 1 mit einem pH-Wert im Bereich von 7,0 bis 8,0.

6. Zusammensetzung nach Anspruch 2, wobei das Tonizitätsmittel in einer ausreichenden Menge vorhanden ist, um Osmolalität von 230 mOsm/kg bis 320 mOsm/kg bereitzustellen.

7. Zusammensetzung nach Anspruch 2, wobei das Tonizitätsmittel in einer Menge im Bereich von 1 % w/v bis 2 % w/v vorhanden ist.

8. Zusammensetzung nach Anspruch 2, wobei das Tonizitätsmittel Propylenglykol ist.

9. Zusammensetzung nach Anspruch 1, ferner umfassend mindestens eine zusätzliche pharmakologisch aktive Verbindung.

10. Zusammensetzung nach Anspruch 9, wobei die mindestens eine zusätzliche pharmakologisch aktive Verbindung fähig ist, eine Augenerkrankung oder -störung zu behandeln, zu lindern und/oder vorzubeugen.

11. Zusammensetzung nach Anspruch 9, wobei die mindestens eine zusätzliche pharmakologisch aktive Verbindung ein Corticosteroid, ein Antihistaminikum, ein Antibiotikum, einen Entzündungshemmer, ein antivirales Medikament, ein Glaukommedikament oder Kombinationen davon ist.

12. Zusammensetzung nach Anspruch 1, ferner umfassend einen Puffer mit einer Konzentration von 20 mM bis 30 mM und 1 % w/v bis 2 % w/v eines Tonizitätsmittels.

13. Zusammensetzung nach Anspruch 1, umfassend 7,5 % w/v Polyvinylpyrrolidon K30 und ferner umfassend einen Natriumdihydrogenphosphat-Monohydrat/Dinatriumhydrogenphosphat-Anhydrat-Puffer mit einer Konzentration von 25 mM und 1,6 % w/v Propylenglykol.

14. Zusammensetzung nach Anspruch 1, wobei die Zusammensetzung eine Verbindung mit der Formel

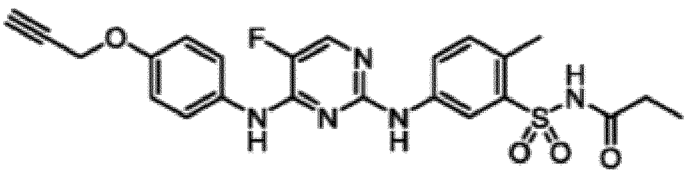

oder ein pharmazeutisch verträgliches Salz, Solvat oder N-Oxid davon umfasst.

**Revendications**

1. Composition pour administration ophtalmique, comprenant :

(i) 5 % p/v à 15 % p/v de polyvinylpyrrolidone ayant un poids moléculaire moyen de 40 000 à 60 000, et moins de 400 ppm de peroxyde ; et
(ii) au moins un composé choisi dans le groupe constitué par

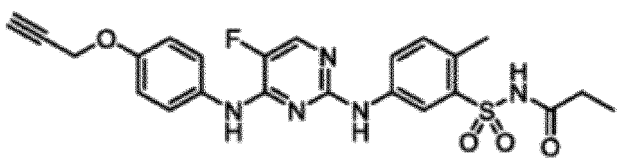
,

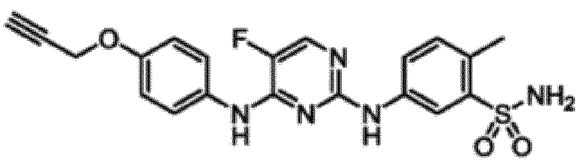
,

et des sels, solvates ou *N*-oxydes pharmaceutiquement acceptables de celui-ci.

**2.** Composition de la revendication 1, comprenant un tampon et un agent de tonicité.

**3.** Composition de la revendication 2, dans laquelle le tampon a une concentration dans la plage d'environ 20 mM à 30 mM.

**4.** Composition de la revendication 3, dans laquelle le tampon est un tampon de phosphate comprenant du monohydrate de phosphate sodique monobasique et un anhydrate de phosphate sodique dibasique.

**5.** Composition de la revendication 1, ayant un pH dans la plage de 7,0 à 8,0.

**6.** Composition de la revendication 2, dans laquelle l'agent de tonicité est présent dans une quantité suffisante pour fournir une osmolalité de 230 mOsm/kg à 320 mOsm/kg.

**7.** Composition de la revendication 2, dans laquelle l'agent de tonicité est présent dans une quantité dans la plage de 1 % p/v à 2 % p/v.

**8.** Composition de la revendication 2, dans laquelle l'agent de tonicité est le propylène glycol.

**9.** Composition de la revendication 1, comprenant en outre au moins un composé pharmacologiquement actif supplémentaire.

**10.** Composition de la revendication 9, dans laquelle l'au moins un composé pharmacologiquement actif supplémentaire est capable de traiter, d'améliorer et/ou de prévenir une maladie ou un trouble oculaire.

**11.** Composition de la revendication 9, dans laquelle l'au moins un composé pharmacologiquement actif supplémentaire est un corticostéroïde, un antihistaminique, un antibiotique, un anti-inflammatoire, un antiviral, une médication pour le glaucome ou des combinaisons de ceux-ci.

**12.** Composition de la revendication 1, comprenant en outre un tampon ayant une concentration de 20 mM à 30 mM, et 1 % p/v à 2 % p/v d'un agent de tonicité.

**13.** Composition de la revendication 1, comprenant 7,5 % p/v de polyvinylpyrrolidone K30, et comprenant en outre un tampon de monohydrate de phosphate sodique monobasique/anhydrate de phosphate sodique dibasique ayant une concentration de 25 mM, et 1,6 % p/v de propylène glycol.

**14.** Composition de la revendication 1, la composition comprenant un composé de formule

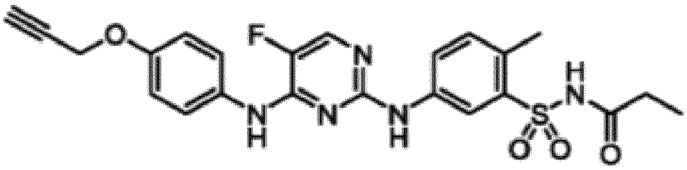

ou un sel, solvate ou N-oxyde pharmaceutiquement acceptable de celui-ci.

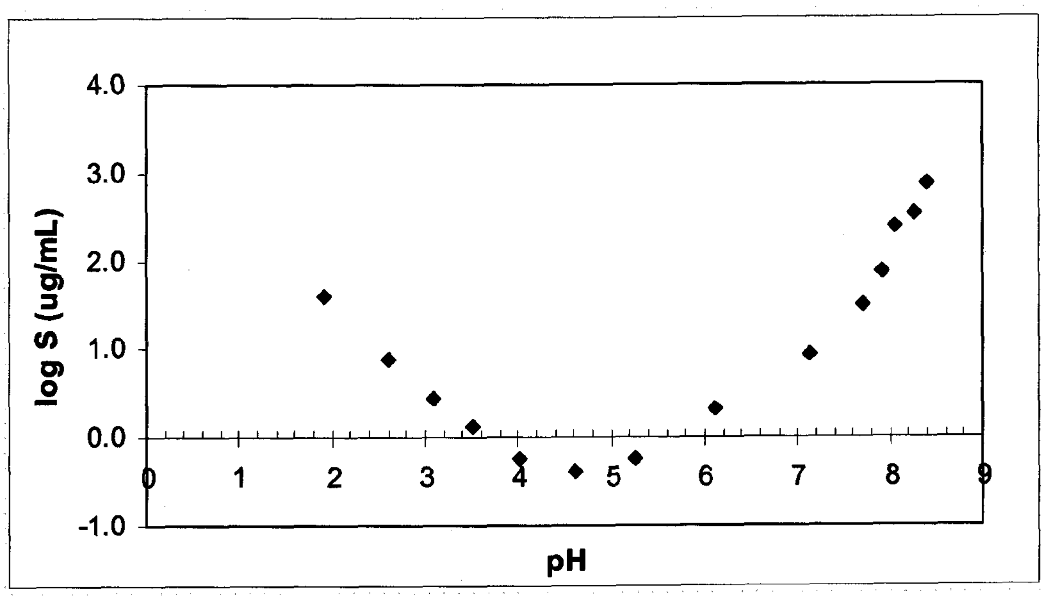
4.0
3.0
2.0
1.0
0.0
-1.0
log S (ug/mL)
0
1
2
3
4
5
6
7
8
9
pH

FIG. 1

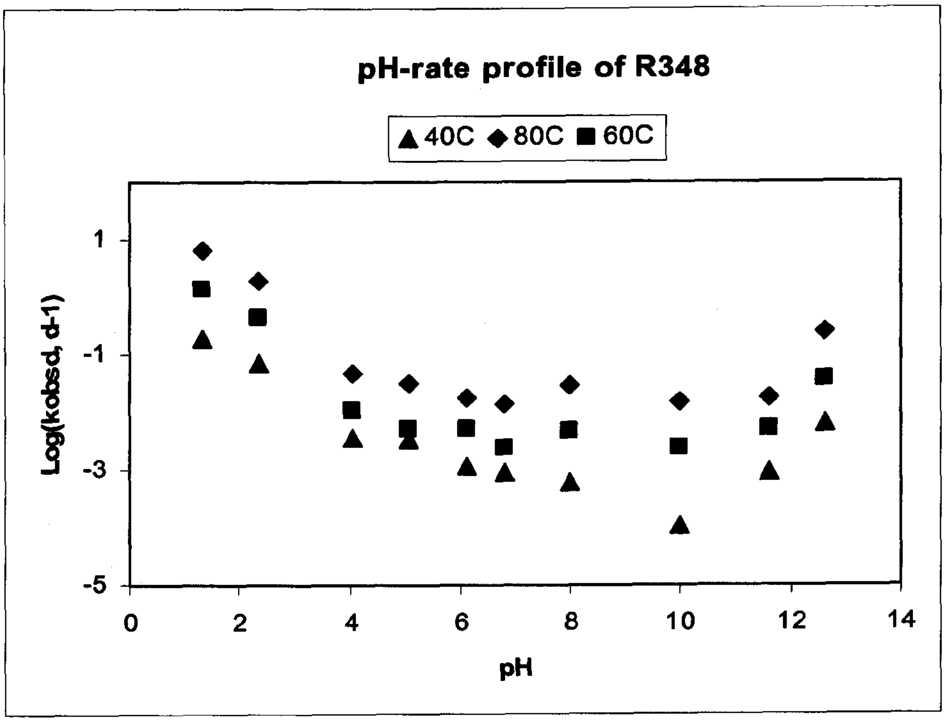
pH-rate profile of R348
▲40C ◆80C ■60C
1
-1
-3
-5
Log(kobsd, d-1)
0
2
4
6
8
10
12
14
pH

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2011028503 A1


### Non-patent literature cited in the description


- **ELIEL, E. L. ; WILEN, S. H.** Stereochemistry of Organic Compounds. Wiley & Sons, 1994
- The Physician's Desk Reference. 2006
- **TAKAHASHI et al.** High incidence of autoimmune dacryoadenitis in male non-obese diabetic (NOD) mice depending on sex steroid. *Clin Exp Immunol.,* 1997, vol. 109, 555-561
- **VAN BLOKLAND ; VERSNEL.** Pathogenesis of Sjögren's syndrome: characteristics of different mouse models for autoimmune exocrinopathy. *Clin Immunol.,* 2002, vol. 103, 111-124